# EUROPEAN PATENT APPLICATION

(11) **EP 3 808 772 A1**
(43) Date of publication of application: **21.04.2021**
(21) Application number: 19796175.8
(22) Date of filing: 02.05.2019
(51) Int. Cl.: C07K 16/28, A61K 47/68

(54) **ANTIBODY SPECIFICALLY BINDING TO C-MET, AND USE THEREOF**

(30) Priority: 02.05.2018 KR 20180050837; 30.04.2019 KR 20190050780
(71) Applicant: AIMED BIO INC., Seoul 05835 (KR)
(72) Inventor: NAM, Do-Hyun, Seoul 06351 (KR); PARK, Hyunkyu, Seoul 06351 (KR); YOON, Yeup, Seoul 06351 (KR)
(74) Representative: Mewburn Ellis LLP
(86) International application number: PCT/KR2019/005257
(87) International publication number: WO 2019/212253

(57) **Abstract**

The present invention relates to an anti-c-Met antibody that is cross-reactive to human and mouse c-Met, and a use thereof and, more specifically, to: an anti-c-Met antibody or an antigen-binding fragment thereof; a bispecific antibody or an antibody-drug conjugate comprising the antibody or an antigen-binding fragment thereof; a pharmaceutical composition for preventing or treating cancer, comprising same; a nucleic acid coding for the antibody or an antigen-binding fragment thereof; a vector and a host cell comprising the nucleic acid; a method for preparing an anti-c-Met antibody or an antigen-binding fragment thereof by using same; and a co-administered cancer treatment composition containing the anti-c-Met antibody or an antigen-binding fragment thereof and another additional cancer treatment agent. An antibody binding to c-Met and an antigen-binding fragment thereof, according to the present invention, can bind to human and mouse c-Met with high affinity, and thus more accurate preclinical results can be identified in an efficacy evaluation by using mouse tumor models. An antibody binding to c-Met and an antigen-binding fragment thereof, according to the present invention, can be effectively used in the prevention or treatment of target cancer.

<110>     SAMSUNG LIFE PUBLIC WELFARE FOUNDATION

<120>     Antibody specifically binding to c-Met and Use thereof

<130>     PP-B2217

<150>     KR 2018-0050837
<151>     2018-05-02

<150>     KR 2019-0050780
<151>     2019-04-30

<160>     69

<170>     KoPatentIn 3.0

<210>     1
<211>     5
<212>     PRT
<213>     Artificial Sequence

<220>
<223>     CDRH1


<400>     1
Asn Tyr Ala Met Ser
  1               5


<210>     2
<211>     17
<212>     PRT
<213>     Artificial Sequence

<220>
<223>     CDRH2


<400>     2
Gly Ile Ser Tyr Ser Gly Gly Ser Thr Tyr Tyr Ala Asp Ser Val Lys
  1               5                  10                  15

Gly



<210>     3
<211>     18
<212>     PRT
<213>     Artificial Sequence

<220>
<223>     CDRH3


<400>     3
Ala Ser Arg Ser Cys Gln Arg Pro Ala Cys Ser Tyr Ala Asn Gly Met
  1               5                  10                  15

Asp Val

<210>    4
<211>    13
<212>    PRT
<213>    Artificial Sequence

<220>
<223>    CDRL1


<400>    4
Thr Gly Ser Ser Ser Asn Ile Gly Asn Asn Tyr Val Thr
 1               5                   10


<210>    5
<211>    7
<212>    PRT
<213>    Artificial Sequence

<220>
<223>    CDRL2


<400>    5
Tyr Asn Asn His Arg Pro Ser
 1               5


<210>    6
<211>    11
<212>    PRT
<213>    Artificial Sequence

<220>
<223>    CDRL3


<400>    6
Gly Ser Trp Asp Tyr Ser Leu Ser Ala Tyr Val
 1               5                   10


<210>    7
<211>    40
<212>    DNA
<213>    Artificial Sequence

<220>
<223>    Primer


<400>    7
ctaccgtggc ccaggcggcc gaggtgcagc tgttggagtc                              40


<210>    8
<211>    39
<212>    DNA
<213>    Artificial Sequence

<220>
<223>    Primer

<400>    8
tggtgctggc cggcctggcc taggaccgtc agcttggtg                              39


<210>    9
<211>    66
<212>    DNA
<213>    Artificial Sequence

<220>
<223>    Primer


<400>    9
tgtgcagcct ctggattcac ctttnnknnk nnknnknnkn nktgggtccg ccaggctcca       60

gggaag                                                                  66


<210>    10
<211>    24
<212>    DNA
<213>    Artificial Sequence

<220>
<223>    Primer


<400>    10
aaaggtgaat ccagaggctg caca                                              24


<210>    11
<211>    75
<212>    DNA
<213>    Artificial Sequence

<220>
<223>    Primer


<400>    11
gggaaggggc tggagtgggt ctcannkatc nnknnknnkn nknnknnknn ktattacgct       60

gattctgtaa aaggt                                                        75


<210>    12
<211>    24
<212>    DNA
<213>    Artificial Sequence

<220>
<223>    Primer


<400>    12
tgagacccac tccagcccct tccc                                              24


<210>    13

<211>    72
<212>    DNA
<213>    Artificial Sequence

<220>
<223>    Primer


<400>    13
atctcttata gtggtggtag tacannknnk nnknnknnkn nknnknnkcg gttcaccatc         60

tccagagaca at                                                             72


<210>    14
<211>    24
<212>    DNA
<213>    Artificial Sequence

<220>
<223>    Primer


<400>    14
tgtactacca ccactataag agat                                               24


<210>    15
<211>    75
<212>    DNA
<213>    Artificial Sequence

<220>
<223>    Primer


<400>    15
gacacggccg tgtattactg tgcgarannk nnkarannkt gtnnknnknn kgcttgttct         60

tatgctaatg gtatg                                                          75


<210>    16
<211>    24
<212>    DNA
<213>    Artificial Sequence

<220>
<223>    Primer


<400>    16
cgcacagtaa tacacggccg tgtc                                                24


<210>    17
<211>    69
<212>    DNA
<213>    Artificial Sequence

<220>
<223>    Primer

<400>    17
gctagtcgtt cttgtcagcg gcctnnktgt nnknnknnkn nknnkatgga cgtctggggc    60

cagggtaca                                                            69


<210>    18
<211>    24
<212>    DNA
<213>    Artificial Sequence

<220>
<223>    Primer


<400>    18
aggccgctga caagaacgac tagc                                           24


<210>    19
<211>    66
<212>    DNA
<213>    Artificial Sequence

<220>
<223>    Primer


<400>    19
gggcagaggg tcaccatctc ttgtnnknnk nnknnknnkn nkattggcaa taattatgtc    60

acctgg                                                               66


<210>    20
<211>    24
<212>    DNA
<213>    Artificial Sequence

<220>
<223>    Primer


<400>    20
acaagagatg gtgaccctct gccc                                           24


<210>    21
<211>    69
<212>    DNA
<213>    Artificial Sequence

<220>
<223>    Primer


<400>    21
tcttgtactg gctcttcatc taatnnknnk nnknnknnkn nknnktggta ccagcagctc    60

ccaggaacg                                                            69

<210>    22
<211>    24
<212>    DNA
<213>    Artificial Sequence

<220>
<223>    Primer


<400>    22
attagatgaa gagccagtac aaga                                              24


<210>    23
<211>    69
<212>    DNA
<213>    Artificial Sequence

<220>
<223>    Primer


<400>    23
acggccccca aactcctcat ctatnnknnk nnknnknnkn nknnkggggt ccctgaccga       60

ttctctggc                                                               69


<210>    24
<211>    24
<212>    DNA
<213>    Artificial Sequence

<220>
<223>    Primer


<400>    24
atagatgagg agtttggggg ccgt                                              24


<210>    25
<211>    107
<212>    DNA
<213>    Artificial Sequence

<220>
<223>    Primer


<400>    25
tggtgctggc cggcctggcc taggaccgtc agcttggtgc ctccgccgaa gacataagca       60

ctcaggctmn nmnnmnnmnn mnnacagtaa taatcagcct catcctc                    107


<210>    26
<211>    92
<212>    DNA
<213>    Artificial Sequence

<220>
<223>    Primer

<400>    26
tggtgctggc cggcctggcc taggaccgtc agcttggtgc ctccgccgaa mnnmnnmnnm        60

nnmnnmnnat aatcccaaga accacagtaa ta                                      92


<210>    27
<211>    5
<212>    PRT
<213>    Artificial Sequence

<220>
<223>    CDRH1


<400>    27
Asn Tyr Ala Met His
1               5


<210>    28
<211>    17
<212>    PRT
<213>    Artificial Sequence

<220>
<223>    CDRH2


<400>    28
Gly Ile Ser Tyr Asp Gly Gly Ser Thr Tyr Tyr Ala Asp Ser Val Lys
1               5                   10                  15

Gly



<210>    29
<211>    17
<212>    PRT
<213>    Artificial Sequence

<220>
<223>    CDRH2


<400>    29
Gly Ile Ser Tyr Ser Gly Gly Ser Lys Tyr Tyr Ala Asp Ser Val Lys
1               5                   10                  15

Gly



<210>    30
<211>    17
<212>    PRT
<213>    Artificial Sequence

<220>
<223>    CDRH2

<400>    30
Gly Ile Ser Tyr Ser Gly Gly Ser Thr Asp Tyr Ala Asp Ser Val Lys
1               5                   10                  15

Gly

<210>    31
<211>    17
<212>    PRT
<213>    Artificial Sequence

<220>
<223>    CDRH2

<400>    31
Gly Ile Ser Tyr Ser Gly Gly Ser Thr Tyr Tyr Asn Asp Ser Val Lys
1               5                   10                  15

Gly

<210>    32
<211>    18
<212>    PRT
<213>    Artificial Sequence

<220>
<223>    CDRH3

<400>    32
Ala Ser Arg Ser Cys Gln Arg Pro Ala Cys His Tyr Ala Asn Gly Met
1               5                   10                  15

Asp Val

<210>    33
<211>    18
<212>    PRT
<213>    Artificial Sequence

<220>
<223>    CDRH3

<400>    33
Ala Ser Arg Ser Cys Gln Arg Pro Ala Cys Ser Tyr Ala Arg Gly Met
1               5                   10                  15

Asp Val

<210>    34
<211>    13

<212>    PRT
<213>    Artificial Sequence

<220>
<223>    CDRL1

<400>    34
Thr Gly Asp Ser Ser Asn Ile Gly Asn Asn Tyr Val Thr
1               5                   10

<210>    35
<211>    13
<212>    PRT
<213>    Artificial Sequence

<220>
<223>    CDRL1

<400>    35
Thr Gly Ser Ser Ser Asp Ile Gly Asn Asn Tyr Val Thr
1               5                   10

<210>    36
<211>    7
<212>    PRT
<213>    Artificial Sequence

<220>
<223>    CDRL2

<400>    36
Glu Asn Asn His Arg Pro Ser
1               5

<210>    37
<211>    7
<212>    PRT
<213>    Artificial Sequence

<220>
<223>    CDRL2

<400>    37
Tyr Asp Asn His Arg Pro Ser
1               5

<210>    38
<211>    11
<212>    PRT
<213>    Artificial Sequence

<220>
<223>    CDRL3

<400>    38
Gly Ser Trp Asp Tyr Ser Leu Arg Ala Tyr Val
1               5                   10

<210>    39
<211>    11
<212>    PRT
<213>    Artificial Sequence

<220>
<223>    CDRL3

<400>    39
Gly Ser Trp Asp Tyr Ser Leu Ser Ala Asp Val
1               5                   10

<210>    40
<211>    127
<212>    PRT
<213>    Artificial Sequence

<220>
<223>    Heavy Chain Variable region

<400>    40
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met His Trp Val Arg Gln Ala Pro Gly Lys Gly Leu Glu Trp Val
        35                  40                  45

Ser Gly Ile Ser Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125

<210>    41
<211>    110
<212>    PRT
<213>    Artificial Sequence

<220>
<223>    Light Chain Variable region

<400>    41
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110


<210>    42
<211>    127
<212>    PRT
<213>    Artificial Sequence

<220>
<223>    Heavy Chain Variable region


<400>    42
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Ser Tyr Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125


<210>    43

<211>    127
<212>    PRT
<213>    Artificial Sequence

<220>
<223>    Heavy Chain Variable region


<400>    43
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Ser Tyr Ser Gly Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125



<210>    44
<211>    127
<212>    PRT
<213>    Artificial Sequence

<220>
<223>    Heavy Chain Variable region


<400>    44
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Ser Tyr Ser Gly Gly Ser Thr Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125

<210>   45
<211>   127
<212>   PRT
<213>   Artificial Sequence

<220>
<223>   Heavy Chain Variable region

<400>   45
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Ser Tyr Ser Gly Gly Ser Thr Tyr Tyr Asn Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125

<210>   46
<211>   127
<212>   PRT
<213>   Artificial Sequence

<220>
<223>   Heavy Chain Variable region

<400>   46
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
            35                  40                  45

Ser Gly Ile Ser Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys His Tyr Ala Asn
            100                 105                 110

Gly Met Asp Val Trp Gly Gln Gly Thr Leu Val Thr Val Ser Ser
        115                 120                 125

<210>    47
<211>    127
<212>    PRT
<213>    Artificial Sequence

<220>
<223>    Heavy Chain Variable region

<400>    47
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
            35                  40                  45

Ser Gly Ile Ser Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Arg
            100                 105                 110

Gly Met Asp Val Trp Gly Gln Gly Thr Leu Val Thr Val Ser Ser
        115                 120                 125

<210>    48
<211>    127
<212>    PRT
<213>    Artificial Sequence

<220>
<223>    Heavy Chain Variable region


<400>    48
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Gly Ile Ser Tyr Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Ser Arg Ser Cys Gln Arg Pro Ala Cys Ser Tyr Ala Asn
            100                 105                 110

Gly Met Asp Val Trp Gly Gln Gly Thr Leu Val Thr Val Ser Ser
        115                 120                 125




<210>    49
<211>    110
<212>    PRT
<213>    Artificial Sequence

<220>
<223>    Light Chain Variable region


<400>    49
Gln Ser Val Leu Thr Gln Pro Pro Ser Ala Ser Gly Thr Pro Gly Gln
1               5                   10                  15

Arg Val Thr Ile Ser Cys Thr Gly Asp Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110

<210>    50
<211>    110
<212>    PRT
<213>    Artificial Sequence

<220>
<223>    Light Chain Variable region


<400>    50
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asp Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110


<210>    51
<211>    110
<212>    PRT
<213>    Artificial Sequence

<220>
<223>    Light Chain Variable region


<400>    51
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Glu Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110

<210>    52
<211>    110
<212>    PRT
<213>    Artificial Sequence

<220>
<223>    Light Chain Variable region


<400>    52
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asp Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110


<210>    53
<211>    110
<212>    PRT
<213>    Artificial Sequence

<220>
<223>    Light Chain Variable region


<400>    53
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Arg Ala Tyr Val Phe Gly Gly Gly Thr Lys Leu Thr Val Leu

100                 105                 110

<210>    54
<211>    110
<212>    PRT
<213>    Artificial Sequence

<220>
<223>    Light Chain Variable region

<400>    54
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Tyr Val Thr Trp Tyr Gln Gln Leu Pro Gly Thr Ala Pro Lys Leu Leu
        35                  40                  45

Ile Tyr Tyr Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Asp Val Phe Gly Gly Gly Thr Lys Leu Thr Val Leu
            100                 105                 110

<210>    55
<211>    381
<212>    DNA
<213>    Artificial Sequence

<220>
<223>    Heavy Chain Variable region

<400>    55
gaggtgcagc tgttggagtc tgggggaggc ttggtacagc ctggggggtc cctgagactc      60

tcctgtgcag cctctggatt cacctttagc aattatgcta tgcactgggt ccgccaggct     120

ccagggaagg ggctggagtg ggtctcaggg atctcttata gtggtggtag tacatattac     180

gctgattctg taaaaggtcg gttcaccatc tccagagaca attccaagaa cacgctgtat     240

ctgcaaatga acagcctgag agccgaggac acggccgtgt attactgtgc gaaagctagt     300

cgttcttgtc agcggcctgc ttgttcttat gctaatggta tggacgtctg gggccagggt     360

acactggtca ccgtgagctc a                                               381

<210>    56
<211>    330
<212>    DNA

<213>    Artificial Sequence

<220>
<223>    Light Chain Variable region


<400>    56
cagtctgtgc tgactcagcc accctcagcg tctgggaccc ccgggcagag ggtcaccatc        60

tcttgtactg gctcttcatc taatattggc aataattatg tcacctggta ccagcagctc       120

ccaggaacgg cccccaaact cctcatctat tataataatc atcggccaag cggggtccct       180

gaccgattct ctggctccaa gtctggcacc tcagcctccc tggccatcag tgggctccag       240

tccgaggatg aggctgatta ttactgtggt tcttgggatt atagcctgag tgcttatgtc       300

ttcggcggag gcaccaagct tacggtccta                                        330


<210>    57
<211>    381
<212>    DNA
<213>    Artificial Sequence

<220>
<223>    Heavy Chain Variable region


<400>    57
gaggtgcagc tgttggagtc tgggggaggc ttggtacagc ctggggggtc cctgagactc        60

tcctgtgcag cctctggatt cacctttagc aattatgcta tgagctgggt ccgccaggct       120

ccagggaagg ggctggagtg ggtctcaggg atctcttatg atggtggtag tacatattac       180

gctgattctg taaaaggtcg gttcaccatc tccagagaca attccaagaa cacgctgtat       240

ctgcaaatga acagcctgag agccgaggac acggccgtgt attactgtgc gaaagctagt       300

cgttcttgtc agcggcctgc ttgttcttat gctaatggta tggacgtctg gggccagggt       360

acactggtca ccgtgagctc a                                                 381


<210>    58
<211>    381
<212>    DNA
<213>    Artificial Sequence

<220>
<223>    Heavy Chain Variable region


<400>    58
gaggtgcagc tgttggagtc tgggggaggc ttggtacagc ctggggggtc cctgagactc        60

tcctgtgcag cctctggatt cacctttagc aattatgcta tgagctgggt ccgccaggct       120

ccagggaagg ggctggagtg ggtctcaggg atctcttata gtggtggtag taaatattac       180

gctgattctg taaaaggtcg gttcaccatc tccagagaca attccaagaa cacgctgtat       240

ctgcaaatga acagcctgag agccgaggac acggccgtgt attactgtgc gaaagctagt 300

cgttcttgtc agcggcctgc ttgttcttat gctaatggta tggacgtctg gggccagggt 360

acactggtca ccgtgagctc a 381

<210> 59
<211> 381
<212> DNA
<213> Artificial Sequence

<220>
<223> Heavy Chain Variable region

<400> 59
gaggtgcagc tgttggagtc tgggggaggc ttggtacagc ctggggggtc cctgagactc 60

tcctgtgcag cctctggatt cacctttagc aattatgcta tgagctgggt ccgccaggct 120

ccagggaagg ggctggagtg ggtctcaggg atctcttata gtggtggtag tacagattac 180

gctgattctg taaaaggtcg gttcaccatc tccagagaca attccaagaa cacgctgtat 240

ctgcaaatga acagcctgag agccgaggac acggccgtgt attactgtgc gaaagctagt 300

cgttcttgtc agcggcctgc ttgttcttat gctaatggta tggacgtctg gggccagggt 360

acactggtca ccgtgagctc a 381

<210> 60
<211> 381
<212> DNA
<213> Artificial Sequence

<220>
<223> Heavy Chain Variable region

<400> 60
gaggtgcagc tgttggagtc tgggggaggc ttggtacagc ctggggggtc cctgagactc 60

tcctgtgcag cctctggatt cacctttagc aattatgcta tgagctgggt ccgccaggct 120

ccagggaagg ggctggagtg ggtctcaggg atctcttata gtggtggtag tacatattac 180

aatgattctg taaaaggtcg gttcaccatc tccagagaca attccaagaa cacgctgtat 240

ctgcaaatga acagcctgag agccgaggac acggccgtgt attactgtgc gaaagctagt 300

cgttcttgtc agcggcctgc ttgttcttat gctaatggta tggacgtctg gggccagggt 360

acactggtca ccgtgagctc a 381

<210> 61
<211> 381
<212> DNA
<213> Artificial Sequence

<220>

<223>    Heavy Chain Variable region

<400>    61
gaggtgcagc tgttggagtc tgggggaggc ttggtacagc ctggggggtc cctgagactc    60

tcctgtgcag cctctggatt cacctttagc aattatgcta tgagctgggt ccgccaggct    120

ccagggaagg ggctggagtg ggtctcaggg atctcttata gtggtggtag tacatattac    180

gctgattctg taaaaggtcg gttcaccatc tccagagaca attccaagaa cacgctgtat    240

ctgcaaatga acagcctgag agccgaggac acggccgtgt attactgtgc gaaagctagt    300

cgttcttgtc agcggcctgc ttgtcattat gctaatggta tggacgtctg gggccagggt    360

acactggtca ccgtgagctc a    381

<210>    62
<211>    381
<212>    DNA
<213>    Artificial Sequence

<220>
<223>    Heavy Chain Variable region

<400>    62
gaggtgcagc tgttggagtc tgggggaggc ttggtacagc ctggggggtc cctgagactc    60

tcctgtgcag cctctggatt cacctttagc aattatgcta tgagctgggt ccgccaggct    120

ccagggaagg ggctggagtg ggtctcaggg atctcttata gtggtggtag tacatattac    180

gctgattctg taaaaggtcg gttcaccatc tccagagaca attccaagaa cacgctgtat    240

ctgcaaatga acagcctgag agccgaggac acggccgtgt attactgtgc gaaagctagt    300

cgttcttgtc agcggcctgc ttgttcttat gctagaggta tggacgtctg gggccagggt    360

acactggtca ccgtgagctc a    381

<210>    63
<211>    381
<212>    DNA
<213>    Artificial Sequence

<220>
<223>    Heavy Chain Variable region

<400>    63
gaggtgcagc tgttggagtc tgggggaggc ttggtacagc ctggggggtc cctgagactc    60

tcctgtgcag cctctggatt cacctttagc aattatgcta tgagctgggt ccgccaggct    120

ccagggaagg ggctggagtg ggtctcaggg atctcttata gtggtggtag tacatattac    180

gctgattctg taaaaggtcg gttcaccatc tccagagaca attccaagaa cacgctgtat    240

ctgcaaatga acagcctgag agccgaggac acggccgtgt attactgtgc gaaagctagt    300

cgttcttgtc agcggcctgc ttgttcttat gctaatggta tggacgtctg gggccagggt 360

acactggtca ccgtgagctc a 381

<210> 64
<211> 330
<212> DNA
<213> Artificial Sequence

<220>
<223> Light Chain Variable region

<400> 64
cagtctgtgc tgactcagcc accctcagcg tctgggaccc ccgggcagag ggtcaccatc 60

tcttgtactg gcgattcatc taatattggc aataattatg tcacctggta ccagcagctc 120

ccaggaacgg cccccaaact cctcatctat tataataatc atcggccaag cggggtccct 180

gaccgattct ctggctccaa gtctggcacc tcagcctccc tggccatcag tgggctccag 240

tccgaggatg aggctgatta ttactgtggt tcttgggatt atagcctgag tgcttatgtc 300

ttcggcggag gcaccaagct tacggtccta 330

<210> 65
<211> 330
<212> DNA
<213> Artificial Sequence

<220>
<223> Light Chain Variable region

<400> 65
cagtctgtgc tgactcagcc accctcagcg tctgggaccc ccgggcagag ggtcaccatc 60

tcttgtactg gctcttcatc tgatattggc aataattatg tcacctggta ccagcagctc 120

ccaggaacgg cccccaaact cctcatctat tataataatc atcggccaag cggggtccct 180

gaccgattct ctggctccaa gtctggcacc tcagcctccc tggccatcag tgggctccag 240

tccgaggatg aggctgatta ttactgtggt tcttgggatt atagcctgag tgcttatgtc 300

ttcggcggag gcaccaagct tacggtccta 330

<210> 66
<211> 330
<212> DNA
<213> Artificial Sequence

<220>
<223> Light Chain Variable region

<400> 66
cagtctgtgc tgactcagcc accctcagcg tctgggaccc ccgggcagag ggtcaccatc 60

tcttgtactg gctcttcatc taatattggc aataattatg tcacctggta ccagcagctc 120

ccaggaacgg cccccaaact cctcatctat gaaaataatc atcggccaag cggggtccct 180

gaccgattct ctggctccaa gtctggcacc tcagcctccc tggccatcag tgggctccag 240

tccgaggatg aggctgatta ttactgtggt tcttgggatt atagcctgag tgcttatgtc 300

ttcggcggag gcaccaagct tacggtccta 330

<210> 67
<211> 330
<212> DNA
<213> Artificial Sequence

<220>
<223> Light Chain Variable region

<400> 67
cagtctgtgc tgactcagcc accctcagcg tctgggaccc ccgggcagag ggtcaccatc 60

tcttgtactg gctcttcatc taatattggc aataattatg tcacctggta ccagcagctc 120

ccaggaacgg cccccaaact cctcatctat tatgataatc atcggccaag cggggtccct 180

gaccgattct ctggctccaa gtctggcacc tcagcctccc tggccatcag tgggctccag 240

tccgaggatg aggctgatta ttactgtggt tcttgggatt atagcctgag tgcttatgtc 300

ttcggcggag gcaccaagct tacggtccta 330

<210> 68
<211> 330
<212> DNA
<213> Artificial Sequence

<220>
<223> Light Chain Variable region

<400> 68
cagtctgtgc tgactcagcc accctcagcg tctgggaccc ccgggcagag ggtcaccatc 60

tcttgtactg gctcttcatc taatattggc aataattatg tcacctggta ccagcagctc 120

ccaggaacgg cccccaaact cctcatctat tataataatc atcggccaag cggggtccct 180

gaccgattct ctggctccaa gtctggcacc tcagcctccc tggccatcag tgggctccag 240

tccgaggatg aggctgatta ttactgtggt tcttgggatt atagcctgag agcttatgtc 300

ttcggcggag gcaccaagct tacggtccta 330

<210> 69
<211> 330
<212> DNA
<213> Artificial Sequence

<220>
<223>    Light Chain Variable region


<400>    69
cagtctgtgc tgactcagcc accctcagcg tctgggaccc ccgggcagag ggtcaccatc        60

tcttgtactg gctcttcatc taatattggc aataattatg tcacctggta ccagcagctc       120

ccaggaacgg cccccaaact cctcatctat tataataatc atcggccaag cggggtccct       180

gaccgattct ctggctccaa gtctggcacc tcagcctccc tggccatcag tgggctccag       240

tccgaggatg aggctgatta ttactgtggt tcttgggatt atagcctgag tgctgatgtc       300

ttcggcggag gcaccaagct tacggtccta                                        330

## Description

### [Technical Field]

The present invention relates to an anti-c-Met antibody that cross-links to human and mouse c-Met and the use thereof, and more particularly to an anti-c-Met antibody or antigen-binding fragment thereof, a bispecific antibody or antibody-drug conjugate including the antibody or antigen-binding fragment thereof, a pharmaceutical composition for preventing or treating cancer containing the same, a nucleic acid encoding the antibody or antigen-binding fragment thereof, a vector and host cell containing the nucleic acid, a method of producing an anti-c-Met antibody or antigen-binding fragment thereof using the same, and a co-administration composition for treating cancer containing the anti-c-Met antibody or antigen-binding fragment thereof and additional therapeutic agent for cancer.

### [Background Art]

Various growth factors such as hepatocyte growth factors (HGFs), epidermal growth factor (EGFs), vascular endothelial growth factors (VEGFs) and fibroblast growth factors (FGFs) interact with receptor tyrosine kinases (RTKs) on the cell surface to induce essential and important cellular physiological regulation, such as cell growth, differentiation, angiogenesis and tissue regeneration, as well as cell generation (cytogenesis). When these growth factors and receptors are deregulated in terms of physiological causes such as mutation, overexpression and self-activation, they cause abnormal cell growth or differentiation, thus initiating and promoting cancer progression (Lemmon M. A. & Schlessinger J., Cell 141: 1117-1134, 2010).

The MET (met proto-oncogene; c-Met) protein is known as a proto-oncogene that expresses a hepatocyte-growth-factor (HGF)/scatter-factor (SF) receptor (Dean M. et al., Nature 318:385-388, 1985, Gherardi et al., Nat. Rev. Cancer 12:89-103, 2012), interacts with the only known ligand, HGF, to induce mesenchymal epithelial transition (MET), and promotes growth, penetration and metastasis of cancer cells. c-Met is considered an effective anticancer target because it is also involved in mechanisms such as generation, metastasis, invasion and angiogenesis, regardless of HGF, a ligand in the development of various tumors. Against this background, c-Met inhibitors such as chemical drugs and monoclonal antibodies are being actively researched (Comoglio PM et al., Nat. Rev. Drug. Discov. 7:504-516, 2008) .

The development of an antagonistic antibody against, the anticancer target, c-Met is a typical anticancer therapy strategy based on inhibition of c-Met. An anti-c-Met antibody has been reported to inhibit the interaction between the ligand HGF and c-Met or to inactivate c-Met through digestion (decomposition). For example, OA-5D5 (one-armed antagonistic antibody) developed as an anti-c-Met antibody is an agonist that has been developed into an antibody modified so as not to have side effects that induce c-Met dimerization (Martens T et al., Clin. Cancer Res. 15:6144-6152, 2006), and DN30 has been developed to induce inactivation of c-Met itself, thereby eliminating the function thereof and inducing the inhibition of tumor formation. (Petrelli A et al., PNAS. 103:5090-5095, 2006). However, a one-arm antagonist antibody has insufficient tumor suppression effects when used alone, but exhibits significant therapeutic effects when used in combination with chemotherapy, and c-Met inactivated antibody was found to have disadvantages of being less competitive with ligands and having a partial effect as an agonist. Therefore, the development of therapeutic antibodies that inhibit the function of human c-Met is still required.

It is necessary to evaluate not only *in-vitro* efficacy but also *in-vivo* preclinical efficacy using a mouse tumor model in order to develop anticancer target antibodies. In particular, the therapeutic efficacy of the corresponding antibody is mainly determined based on preclinical results such as the ability to reduce tumor size and increase the number of days of survival, which can be detected when evaluating the efficacy using a mouse tumor model. The mouse tumor model used at this time is constructed by injecting human-derived cancer cells that overexpress anticancer targets. There is a high probability that the correlation between preclinical results and clinical results when the therapeutic effects of antibodies are determined will be low due to interference not only from injected human tumor cells but also from mixed mouse-derived cells in an actual tumor microenvironment in mice (Talmadge J.E. et al., Am. J. Pathol. 170:793-804, 2007). Therefore, antibodies inhibiting human-derived anticancer targets, combinatorial treatment thereof with antibodies inhibiting mouse-derived anticancer targets or ligands, or antibodies specific for human/mouse xenogeneic anticancer targets may exhibit more accurate preclinical treatment results in tumor models. For example, an anti-Dll4 (delta-like ligand 4) antibody that inhibits angiogenesis in tumors was reported to significantly reduce the size of the tumor through combinatorial treatment of an antibody against mouse Dll4 and an antibody against human Dll4 in a mouse tumor model (Hoey T. et al., Cell Stem Cell. 5:168-177, 2009). In addition, in the case of antibodies targeting vascular endothelial growth factor 2 (VEGFR-2) or vascular endothelial growth factor (VEGF), as well, antibodies that are cross-reactive to human/mouse xenogeneic anticancer targets exhibit high tumor suppression effects in mouse tumor models, which suggests the need for development of cross-reactive antibodies (Huang J. et al., Cytotechnology 62:61-71, 2010; Liang W-C et al., J. Biol. Chem. 281:951-961, 2006).

As described above, anti-c-Met antibodies that inhibit only the function of c-Met have no inhibitory effect against mouse c-Met receptors regarding the autocrine/paracrine action of human- or mouse-derived hepatocyte growth factors. For this reason, it is difficult to evaluate preclinical efficacy and effects in a mouse tumor model. Human c-Met (P08581, UniProtKB/Swiss-Prot) is composed of 1,390 amino acids and mouse c-Met (P16056, UniProtKB/Swiss-Prot) is composed of 1,379 amino acids, so human c-Met and mouse c-Met have high sequence homology of 89% or more therebetween (Chan AML et al., Oncogene. 2:593-599, 1988) . Hepatocyte growth factor, a ligand, also has very high sequence homology of 90% or more between humans and mice (Tashiro K et al., PNAS. 87:3200-3204, 1990), and the typical sites where ligands and receptors act are Sema domains and thus the possibility of development and application of cross-reactive antibodies is high. Therefore, there is need for the development of an antibody that cross-reacts with human/mouse c-Met that can exhibit results of effective preclinical research in a mouse tumor model by inhibiting the action of cancer-specific ligand receptors on human/mouse c-Met in antibody tumor microenvironments.

Against this technical background, the present inventors developed an improved antibody having the ability to cross-link to human and mouse c-Met and improved ability to bind to c-Met using an affinity maturation process, and thereby completed the present invention.

The information disclosed in this Background section is provided only for better understanding of the background of the present invention, and therefore it may not include information that forms the prior art that is already obvious to those skilled in the art.

### [Disclosure]

Therefore, the present invention has been made in view of the above problems, and it is one object of the present invention to provide an anti-c-Met antibody or antigen-binding fragment thereof that specifically binds to c-Met.

It is another object of the present invention to provide a bispecific antibody or antibody-drug conjugate including the anti-c-Met antibody or antigen-binding fragment thereof.

It is another object of the present invention to provide a pharmaceutical composition for preventing or treating cancer, containing the anti-c-Met antibody or antigen-binding fragment thereof and/or the bispecific antibody or antibody-drug conjugate, and a method of treating cancer using the pharmaceutical composition.

It is another object of the present invention to provide a nucleic acid encoding the anti-c-Met antibody or antigen-binding fragment thereof, a vector and host cell containing the nucleic acid, and a method of producing an anti-c-Met antibody or antigen-binding fragment thereof using the same.

It is another object of the present invention to provide a co-administration composition for treating cancer containing the anti-c-Met antibody or antigen-binding fragment thereof and an additional therapeutic agent for cancer and a method of treating cancer using the same.

It is another object of the present invention to provide the use of the antibody or antigen-binding fragment thereof, or the bispecific antibody or antibody-drug conjugate for the treatment of cancer.

It is another object of the present invention to provide the use of the antibody or antigen-binding fragment thereof, or the bispecific antibody or antibody-drug conjugate for the preparation of a therapeutic agent for cancer.

In accordance with one aspect of the present invention, the above and other objects can be accomplished by the provision of an anti-c-Met antibody or antigen-binding fragment thereof including: a heavy-chain variable region including a heavy chain CDR1 having an amino acid sequence of SEQ ID NO: 1 or 27, a heavy chain CDR2 having an amino acid sequence selected from the group consisting of SEQ ID NOS: 2 and 28 to 31 and a heavy chain CDR3 having an amino acid sequence selected from the group consisting of SEQ ID NOS: 3, 32 and 33; and a light-chain variable region including a light chain CDR1 having an amino acid sequence selected from the group consisting of SEQ ID NOS: 4, 34 and 35, a light chain CDR2 having an amino acid sequence selected from the group consisting of SEQ ID NOS: 5, 36 and 37 and a light chain CDR3 having an amino acid sequence selected from the group consisting of SEQ ID NOS: 6, 38 and 39.

In accordance with another aspect of the present invention, there is provided a bispecific antibody or antibody-drug conjugate including the anti-c-Met antibody or antigen-binding fragment thereof.

In accordance with another aspect of the present invention, there are provided a pharmaceutical composition for preventing or treating cancer, containing the anti-c-Met antibody or antigen-binding fragment thereof and/or the bispecific antibody or antibody-drug conjugate, and a method of treating cancer using the same.

In accordance with another aspect of the present invention, there are provided a nucleic acid encoding the anti-c-Met antibody or antigen-binding fragment thereof, a vector and host cell containing the nucleic acid, and a method of producing an anti-c-Met antibody or antigen-binding fragment thereof using the same.

In accordance with another aspect of the present invention, there are provided a co-administration composition for treating cancer containing the anti-c-Met antibody or antigen-binding fragment thereof and an additional therapeutic agent for cancer, and a method of treating cancer using the same.

In accordance with another aspect of the present invention, there is provided a method of treating cancer including administering a co-administration composition containing the antibody or antigen-binding fragment thereof, or the bispecific antibody or antibody-drug conjugate.

In accordance with another aspect of the present invention, there is provided the use of the antibody or antigen-binding fragment thereof, or the bispecific antibody or antibody-drug conjugate for the treatment of cancer, and the use of the antibody or antigen-binding fragment thereof, or the bispecific antibody or antibody-drug conjugate for the preparation of a therapeutic agent for cancer.

### [Description of Drawings]

FIG. 1 shows a heavy-chain variable-region sequence and CDR/framework classification of a parent antibody (1F12).
FIG. 2 shows a light-chain variable-region sequence and CDR/framework classification of the parent antibody (1F12).
FIG. 3 is a design of a mutant library of an anti-c-Met antibody.
FIG. 4 shows primer sequences for constructing the mutant library of the anti-c-Met antibody.
FIG. 5 shows CDR sequences of anti-c-Met antibody affinity variants.
FIG. 6 shows the results of analysis of agonist activity of 16 anti-c-Met antibody affinity variants.
FIG. 7 shows the results of analysis of affinity of anti-c-Met antibodies.
FIG. 8 shows the results of analysis of the growth inhibition pattern in gastric cancer cell lines.
FIG. 9 shows the results of analysis of the efficacy of the anti-c-Met antibody alone and a combination of the anti-c-Met antibody with an immunotherapy agent in colon cancer cell lines.
FIG. 10 shows the results of analysis of the efficacy of a combination of the anti-c-Met antibody with radiation therapy in colon cancer cell lines.
FIG. 11 shows the results of analysis of the distribution of immune cells in a subcutaneous colon-cancer implant animal model.
FIG. 12 shows the change in expression of PD-L1 in tumor cells after administration of an anti-c-Met antibody in a subcutaneous colon-cancer implant animal model.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

The application of antibody engineering technologies is required in order to improve antibody efficacy. Among them, affinity maturation matures the affinity of an antibody for an antigen. Affinity maturation refers to a method of increasing the binding affinity of an antibody to an antigen by introducing a random mutation into an antibody gene, and is very useful for the development of novel effective therapeutic and diagnostic antibody drugs. For *in-vitro* affinity maturation, three approaches are generally used. These approaches include error-prone PCR, randomization of target residues using degenerated oligonucleotides, and chain shuffling. The portions that can be selected as target residues are complementarity-determining regions (CDR), in particular, CDR-H3 and CDR-L3, which tend to dominate antibody-antigen interactions and thus are logical targets for randomization. The binding affinity of the antibody is improved by changing the amino acid in the target CDR region of the antibody gene. This method was reported to increase the binding affinity up to 22 times by changing the amino acid in CDR-H3 of AKA (a humanized antibody that binds to tumor-associated glycoprotein 72) (Hong et al., J. Biol. Chem. 2006, 281, 6985-6992), and antibodies developed for the hepatitis B virus antigen have also been reported to increase the binding affinity up to 6 times (Hong el al., J. Microbiol. 2007, 45, 528-533).

In one aspect, the present invention is directed to an anti-c-Met antibody or antigen-binding fragment thereof, preferably an anti-c-Met antibody or antigen-binding fragment thereof including: a heavy-chain variable region including a heavy chain CDR1 having an amino acid sequence of SEQ ID NO: 1 or 27, a heavy chain CDR2 having an amino acid sequence selected from the group consisting of SEQ ID NOS: 2 and 28 to 31 and a heavy chain CDR3 having an amino acid sequence selected from the group consisting of SEQ ID NOS: 3, 32 and 33; and a light-chain variable region including a light chain CDR1 having an amino acid sequence selected from the group consisting of SEQ ID NOS: 4, 34 and 35, a light chain CDR2 having an amino acid sequence selected from the group consisting of SEQ ID NOS: 5, 36 and 37 and a light chain CDR3 having an amino acid sequence selected from the group consisting of SEQ ID NOS: 6, 38 and 39.

According to the present invention, an antibody or antigen-binding fragment thereof binding to c-met, which includes a heavy-chain variable region having a sequence having at least 80% sequence homology, preferably at least 90% sequence homology, and more preferably 99% sequence homology with the heavy chain CDR1 having an amino acid sequence of SEQ ID NO: 1 or 27, the heavy chain CDR2 having an amino acid sequence selected from the group consisting of SEQ ID NOS: 2 and 28 to 31, and the heavy chain CDR3 having an amino acid sequence selected from the group consisting of SEQ ID NOS: 3, 32 and 33, and has the same characteristics as c-Met according to the present invention also falls within the scope of the anti-c-Met antibody or antigen-binding fragment thereof according to the present invention.

According to the present invention, an antibody or antigen-binding fragment thereof, which includes a light-chain variable region having a sequence having at least 80% sequence homology, preferably at least 90% sequence homology, and more preferably 99% sequence homology with the light chain CDR1 having an amino acid sequence selected from the group consisting of SEQ ID NOS: 4, 34 and 35; the light chain CDR2 having an amino acid sequence selected from the group consisting of SEQ ID NOS: 5, 36 and 37; and the light chain CDR3 having an amino acid sequence selected from the group consisting of SEQ ID NOS: 6, 38 and 39, and has the same characteristics as c-Met according to the present invention, also falls within the scope of the anti-c-Met antibody or antigen-binding fragment thereof according to the present invention.

According to the present invention, the anti-c-Met antibody or antigen-binding fragment thereof may include a heavy-chain variable region including an amino acid sequence selected from the group consisting of SEQ ID NOS: 40 and 42 to 48, and a light-chain variable region selected from the group consisting of SEQ ID NOS: 41 and 49 to 54.

Meanwhile, an antibody or antigen-binding fragment thereof, which has a sequence having at least 80% sequence homology, preferably at least 90% sequence homology, and more preferably 99% sequence homology with the heavy-chain variable region including an amino acid sequence selected from the group consisting of SEQ ID NOS: 40 and 42 to 48, and has the same characteristics as c-Met according to the present invention, also falls within the scope of the anti-c-Met antibody or antigen-binding fragment thereof according to the present invention.

Meanwhile, an antibody or antigen-binding fragment thereof, which includes a light-chain variable region having a sequence having at least 80% sequence homology, preferably at least 90% sequence homology, more preferably 99% sequence homology with the light-chain variable region including an amino acid sequence selected from the group consisting of SEQ ID NOS: 41 and 49 to 54, and has the same characteristics as c-Met according to the present invention, also falls within the scope of the anti-c-Met antibody or antigen-binding fragment thereof according to the present invention.

In addition, the anti-c-Met antibody or antigen-binding fragment thereof according to the present invention also includes an antibody or antigen-binding fragment thereof in which a part of the amino acid sequence in the anti-c-Met antibody or antigen-binding fragment thereof according to the present invention is substituted through conservative substitution.

As used herein, the term "conservative substitution" refers to modification of a polypeptide including substitution of one or more amino acids with amino acids having similar biochemical properties that do not cause loss of biological or biochemical function of the polypeptide. The term "conservative amino acid substitution" refers to substitution of amino acid residues with amino acid residues having similar side chains thereto. Classes of amino acid residues having similar side chains are defined and are well known in the art. These classes include amino acids having basic side chains (e.g. lysine, arginine, histidine), amino acids having acidic side chains (e.g. aspartic acid, glutamic acid), amino acids having uncharged polar side chains (e.g. glycine, asparagine, glutamine, serine, threonine, tyrosine, cysteine), amino acids having nonpolar side chains (e.g., alanine, valine, leucine, isoleucine, proline, phenylalanine, methionine, tryptophan), amino acids having beta-branched side chains (e.g., threonine, valine, isoleucine) and amino acids having aromatic side chains (e.g., tyrosine, phenylalanine, tryptophan, histidine). It is expected that the antibodies of the present invention have conservative amino acid substitutions and are still active.

As herein used, the term "c-Met-specific antibody" refers to an antibody that binds to c-Met and inhibits the biological activity of c-Met, and is used interchangeably with "anti-c-Met antibody".

As used herein, the term "anti-c-Met antibody" includes both a polyclonal antibody and a monoclonal antibody, but is preferably a monoclonal antibody, and may have an intact whole antibody form. The whole antibody has a structure having two full-length light chains and two full-length heavy chains, and has a structure including a constant region, and each light chain is connected to a heavy chain by a disulfide bond.

The whole antibody of the anti-c-Met antibody according to the present invention includes IgA, IgD, IgE, IgM and IgG forms, and IgG includes subtypes of IgG1, IgG2, IgG3 and IgG4.

The anti-c-Met antibody according to the present invention is preferably a fully human antibody selected from human antibody libraries, but is not limited thereto.

As herein used, the term "antigen-binding fragment" of the anti-c-Met antibody according to the present invention means a fragment having the function of binding to an antigen of the anti-c-Met antibody, that is, c-Met, includes Fab, Fab', F(ab')₂, scFv, (scFv)₂, scFv-Fc, Fv and the like, and is herein used interchangeably with "antibody fragment" having the same meaning.

Fab refers to a structure including a variable domain of each of the heavy chain and the light chain, the constant domain of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. A F(ab')₂ antibody is created by a disulfide bond between cysteine residues in the hinge region of Fab'.

The Fv (variable fragment) is the minimal antibody fragment having only a heavy-chain variable domain and a light-chain variable domain. A two-chain Fv (dsFv, disulfide-stabilized Fv) is a fragment in which the variable domain of the heavy chain and the variable domain of the light chain are linked by a disulfide bond, and a single-chain Fv (scFv) is a fragment in which the variable domain of the heavy chain and the variable domain of the light chain are typically linked by a covalent bond via a peptide linker. Such antibody fragments may be obtained using proteases (e.g., Fab can be obtained by cleaving the whole antibody with papain, and the F(ab')₂ fragment can be obtained by cleaving the whole antibody with pepsin), and may be prepared using genetic recombination technology (for example, DNA encoding the heavy chain of an antibody or variable region thereof, and DNA encoding the light chain or variable region thereof as templates are amplified by PCR (polymerase chain reaction) using a pair of primers, and a combination of DNA encoding a peptide linker and a pair of primers that have both ends connected to the heavy chain or a variable region thereof and the light chain or a variable region thereof, respectively, is amplified).

As used herein, the term "heavy chain" encompasses both a full-length heavy chain, which includes a variable domain (VH) containing an amino acid sequence having a variable-region sequence sufficient for imparting specificity to an antigen and three constant domains (CH1, CH2 and CH3), and a fragment thereof. As used herein, the term "light chain" encompasses both a full-length light chain, which includes a variable domain (VL) containing an amino acid sequence having a variable-region sequence sufficient for imparting specificity to an antigen and a constant domain (CL), and a fragment thereof.

As used herein, the term "complementarity-determining region" (CDR) refers to an amino acid sequence of a hypervariable region of immunoglobulin heavy and light chains (Kabat et al., Sequences of Proteins of Immunological Interest, 4th Ed., U.S. Department of Health and Human Services, National Institutes of Health (1987)). The heavy chain (CDRH1, CDRH2 and CDRH3) and the light chain (CDRL1, CDRL2 and CDRL3) each contain three CDRs. CDRs provide contact residues essential for binding antibodies to antigens or epitopes.

According to the present invention, the anti-c-Met antibody or antigen-binding fragment thereof may have specific binding ability for human c-Met. Preferably, the anti-c-Met antibody or antigen-binding fragment thereof may have cross-linking ability to human c-Met and mouse c-Met, but the present invention is not limited thereto.

In another aspect, the present invention is directed to an antibody-drug conjugate (ADC) including a drug and the anti-c-Met antibody or antigen-binding fragment thereof conjugated to each other.

The antibody-drug conjugate (ADC) requires that the anticancer drug should stably bind to the antibody until the anticancer drug is delivered to the target cancer cell. The drug delivered to the target should be released from the antibody to thereby induce the death of the target cell. For this purpose, the drug should stably bind to the antibody, and should have sufficient cytotoxicity to induce the death of the target cell when released from the target cell.

According to the present invention, the anti-c-Met antibody or an antigen-binding fragment thereof and a cytotoxic substance including a drug such as an anti-cancer agent are bound to each other (for example, via a covalent bond, a peptide bond or the like) and thus can be used as a conjugate or a fusion protein (when a cytotoxic substance and/or labeling substance (marker) is a protein). The cytotoxic substance may be any substance which is toxic to cancer cells, particularly, to solid cancer cells, and may include at least one selected from the group consisting of radioisotopes, cytotoxic compounds (small molecules), cytotoxic proteins and anticancer drugs, but the present invention is not limited thereto. The cytotoxic protein may at least one selected from the group consisting of ricin, saporin, gelonin, momordin, debouganin, diphtheria toxin, pseudomonas toxin, and the like, but the present invention is not limited thereto. The radioisotope may include at least one selected from the group consisting of 1311, 188Rh and 90Y, but the present invention is not limited thereto. The cytotoxic compound may include at least one selected from the group consisting of duocarmycin, monomethyl auristatin E (MMAE), monomethyl auristatin F (MMAF), N2'-diacetyl-N2'-(3-mercapto-1-oxopropyl)maytansine (DM1), PBD (pyrrolobenzodiazepine) dimers, and the like, but is not limited thereto.

According to the present invention, the antibody-drug conjugate may be obtained according to a method well-known in the art.

According to the present invention, the antibody-drug conjugate may be characterized in that the antibody or antigen-binding fragment thereof is bound to the drug via a linker.

According to the present invention, the linker may be a cleavable linker or a non-cleavable linker.

The linker is a means for linking the anti-c-Met antibody to the drug. For example, the linker allows the drug to be released from the antibody in a cleavable form under intracellular conditions, that is, through cleavage of the linker in an intracellular environment.

The linker may be a peptide linker that can be cleaved by a cleavage agent present in an intracellular environment, for example, in the lysosome or endosome, and can be cleaved by intracellular peptidases or proteases, such as lysosome or endosome proteases. Generally, a peptide linker has an amino acid length of at least two. The cleavage agent may include cathepsin B, cathepsin D and plasmin, which hydrolyze the peptide to release the drug into the target cell. The peptide linker can be cleaved by a thiol-dependent protease cathepsin-B, which is strongly expressed in cancer tissues, and may, for example, be a Phe-Leu or Gly-Phe-Leu-Gly linker. In addition, the peptide linker may, for example, be a Val-Cit linker or a Phe-Lys linker, which can be cleaved by an intracellular protease.

According to the present invention, the cleavable linker is sensitive to pH and may be sensitive to hydrolysis at a certain pH value. Generally, the pH-sensitive linker is a linker that can be hydrolyzed under acidic conditions. Examples of acid-instable linkers that can be hydrolyzed in lysosomes may include hydrazone, semicarbazone, thiosemicarbazone, cis-aconitic amide, orthoester, acetal, ketal, and the like.

The linker may also be cleaved under reducing conditions, and may, for example, be a disulfide linker. A variety of disulfide bonds can be formed using N-succinimidyl-S-acetylthioacetate (SATA), N-succinimidyl-3-(2-pyridyldithio) propionate (SPDP), N-succinimidyl-3-(2-pyridyldithio)butyrate (SPDB) and N-succinimidyl-oxycarbonyl-alpha-methyl-alpha-(2-pyridyl-dithio)toluene (SMPT).

According to the present invention, the drug and/or drug linker may be randomly conjugated through the lysine of the antibody, or may be conjugated through cysteine, which is exposed when the disulfide bond chain is reduced. In some cases, the drug-linker can be conjugated through cysteine present in a genetically engineered tag, e.g., a peptide or protein. The genetically engineered tag, e.g., peptide or protein, may include an amino acid motif that can be recognized, for example, by an isoprenoid transferase. The peptide or protein has a deletion at the carboxyl terminus of the peptide or protein or an addition at the carboxyl (C) terminus of the peptide or protein through a covalent bond of the spacer unit. The peptide or protein may be directly covalently bonded to an amino acid motif, or may be linked to the amino acid motif through a covalent bond with the spacer unit. The amino acid spacer unit includes 1 to 20 amino acids, and is preferably a glycine unit among them.

The linker may include a beta-glucuronide linker that is present in multiple copies in the lysosome, or is recognized and hydrolyzed by beta-glucuronidase that is overexpressed in some tumor cells. Unlike the peptide linker, this linker has an advantage of increasing the solubility of the antibody-drug composite when bound to a drug having high hydrophobicity due to the high hydrophilicity thereof.

In this regard, according to the present invention, the beta-glucuronide linker disclosed in Korean Patent Laid-Open No. 2015-0137015, for example, a beta-glucuronide linker including a self-immolative group, may be used.

In addition, the linker can be, for example, a non-cleavable linker, and the drug may be capable of being released merely through a single step of hydrolyzing the antibody to produce, for example, an amino acid-linker-drug composite. This type of linker may be a thioether group or a maleimidocaproyl group, and can remain stable in the blood.

According to the present invention, the drug may be a chemotherapeutic agent, toxin, microRNA (miRNA), siRNA, shRNA or a radioactive isotope. The drug, as an agent having a pharmacological effect, may be conjugated to an antibody.

The chemotherapeutic agent may be a cytotoxic agent or an immunosuppressive agent. Specifically, the chemotherapeutic agent may include a microtubulin inhibitor, a mitotic inhibitor, a topoisomerase inhibitor, or a chemotherapeutic agent capable of functioning as a DNA intercalator. The chemotherapeutic agent may also include an immunomodulatory compound, an anticancer agent, an antiviral agent, an antibacterial agent, an antifungal agent, an anthelmintic agent or a combination thereof.

For example, the drug may include at least one selected from the group consisting of maytansinoid, auristatin, aminopterin, actinomycin, bleomycin, thalidomide, camptothecin, N8-acetylspermidine, 1-(2 chloroethyl)-1,2-dimethyl sulfonyl hydrazide, esperamycin, etoposide, 6-mercaptopurine, dolastatin, trichothecene, calicheamicin, taxol, taxane, paclitaxel, docetaxel, methotrexate, vincristine, vinblastine, doxorubicin, melphalan, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin (mitomycin A, mitomycin C), dacarbazine, procarbazine, topotecan, nitrogen mustard, cytoxan, 5-fluorouracil, bis-chloroethylnitrosourea (CNU), irinotecan, camptothecin, idarubicin, daunorubicin, dactinomycin, plicamycin, asparaginase, vinorelbine, chlorambucil, melphalan, carmustine, lomustine, busulfan, treosulfan, decarbazine, teniposide, topotecan, 9-aminocamptothecin, crisnatol, trimetrexate, mycophenolic acid, tiazofurin, ribavirin, 5-ethynyl-1-beta-Dribofuranosylimidazole-4-carboxamide (EICAR), hydroxyurea, deferoxamine, floxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-a, interferon-γ, tumor necrosis factor, gemcitabine, velcade, revamid, lovastatin, 1-methyl-4-phenylpyridiniumion, staurosporine, actinomycin D, dactinomycin, bleomycin A2, bleomycin B2, peplomycin, epirubicin, pirarubicin, zorubicin, mitoxantrone, verapamil and thapsigargin, nucleases and toxins derived from bacteria or plants and animals, but is not limited thereto.

According to the present invention, the drug may have at least one nucleophile group selected from the group consisting of amine, thiol, hydroxyl, hydrazide, oxime, hydrazine, thiosemicarbazone, hydrazine carboxylate and aryl hydrazide groups, which can react with an electrophilic group on the linker and the linker reagent to form a covalent bond.

In another aspect, the present invention is directed to a bispecific antibody including the anti-c-Met antibody or an antigen-binding fragment thereof.

The bispecific antibody refers to an antibody in which, among two arms of the antibody, one arm includes the anti-c-Met antibody or an antigen-binding fragment thereof according to the present invention, and the other includes an antibody that is specific for an antigen other than the c-Met, preferably a cancer-associated antigen or an immune checkpoint protein antigen, or an antibody which specifically binds to an immune-effector cell-associated antigen, or an antigen-binding fragment thereof.

The antigen, to which the antibody binds, other than the anti-c-Met antibody included in the bispecific antibody according to the present invention is preferably selected from cancer-associated antigens or immune checkpoint protein antigens including HGF, EGFR, EGFRvIII, Her2, Her3, IGF-1R, VEGF, VEGFR-1, VEGFR-2, VEGFR-3, Ang2, Dll4, NRP1, FGFR, FGFR2, FGFR3, c-Kit, MUC1, MUC16, CD20, CD22, CD27, CD30, CD33, CD40, CD52, CD70, CD79, DDL3, Folate R1, Nectin 4, Trop2, gpNMB, Axl, BCMA, PD-1, PD-L1, PD-L2, CTLA4, BTLA, 4-1BB, ICOS, GITR, OX40, VISTA, TIM-3, LAG-3, KIR, B7.1, B7.2, B7-H2, B7-H3, B7-H4, B7-H6, B7-H7, EphA2, EphA4, EphB2, E-selectin, EpCam, CEA, PSMA, PSA, and c-MET, and is preferably selected from immune-effector cell-associated antigens including TCR/CD3, CD16(FcγRIIIa) CD44, CD56, CD69, CD64(FcγRI), CD89, CD11b/CD18(CR3) and the like, but the present invention is not limited thereto.

In another aspect, the present invention is directed to a pharmaceutical composition for preventing and/or treating cancer including the anti-c-Met antibody or an antigen-binding fragment thereof.

In another aspect, the present invention is directed to a pharmaceutical composition for preventing and/or treating cancer including the bispecific antibody or the antibody-drug conjugate.

According to the present invention, the cancer may be associated with expression or overexpression of c-Met.

According to the present invention, the terms "cancer" and "tumor" are used with the same meaning, and refer to or mean the physiological condition of a mammal characterized by uncontrolled cell growth/proliferation.

According to the present invention, the anti-c-Met antibody inhibits the growth of cancer cells derived from various carcinomas due to inhibition of strong anti-c-Met binding and thus c-Met function caused thereby, and inhibits phosphorylation of c-Met and downstream signal transducers to thereby inhibit c-Met signaling and angiogenesis. Therefore, the antibody of the present invention is very effective for the prevention and treatment of cancer.

The cancer or carcinoma that can be treated with the composition of the present invention is not particularly limited, and includes both solid cancer and blood cancer. Examples of such cancer include, but are not limited to, breast cancer, colon cancer, lung cancer, stomach cancer, liver cancer, blood cancer, bone cancer, pancreatic cancer, skin cancer, brain cancer, uterine cancer, nasopharyngeal cancer, laryngeal cancer, colon cancer, ovarian cancer, rectal cancer, colorectal cancer, vaginal cancer, small-intestine cancer, endocrine cancer, thyroid cancer, parathyroid cancer, ureteral cancer, urethral cancer, prostate cancer, bronchial cancer, bladder cancer, kidney cancer and bone-marrow cancer. The cancer may be a primary cancer or a metastatic cancer. More preferably, the cancer that can be prevented or treated by the pharmaceutical composition may be c-Met expression cancer.

According to the present invention, the pharmaceutical composition may be used in combination therapy using radiation.

In another aspect, the present invention is directed to a method for preventing and/or treating a c-Met-associated disease including administering a therapeutically effective amount of the anti-c-Met antibody or antigen-binding fragment thereof and/or the bispecific antibody or the antibody-drug conjugate to a patient in need of preventive or therapeutic treatment for c-Met-associated disease. The method for preventing and/or treating the disease may further include identifying the patient in need of preventive or therapeutic treatment for the disease before administration.

According to the present invention, the method of treatment includes administering a pharmaceutical composition containing the anti-c-Met antibody or antigen-binding fragment thereof and performing irradiation.

The radiation may be applied in a dose of 2Gy to 10Gy, but is not limited thereto.

In the method of the present invention, the number of times of irradiation, the time interval between irradiation, and the administration of the pharmaceutical composition may vary according to the method of the present invention, but preferably, the irradiation may be conducted simultaneously with administration of the pharmaceutical composition, or may be conducted 10 to 20 days after administration of the pharmaceutical composition, but is not limited thereto.

In another aspect, the present invention is directed to the use of the antibody or antigen-binding fragment thereof, or the bispecific antibody or antibody-drug conjugate for the treatment of cancer.

In another aspect, the present invention is directed to the use of the antibody or antigen-binding fragment thereof, or the bispecific antibody or antibody-drug conjugate for the preparation of a therapeutic agent for cancer.

In the pharmaceutical composition, treatment method and use according to the present invention, the anti-c-Met antibody or antigen-binding fragment thereof is provided as an active ingredient alone, or is administered in combination with a cytotoxic substance such as an anticancer agent, or is provided in the form of an antibody-drug conjugate (ADC) conjugated with a toxic substance.

The anti-c-Met antibody or antigen-binding fragment thereof and the pharmaceutical composition containing the same according to the present invention may be used in combination with a conventional therapeutic agent. That is, the anti-c-Met antibody or antigen-binding fragment thereof and the pharmaceutical composition containing the same according to the present invention may be administered simultaneously or sequentially with a conventional anticancer agent.

According to the present invention, the pharmaceutical composition may include a therapeutically effective amount of the anti-c-Met antibody or antigen-binding fragment thereof, and a pharmaceutically acceptable carrier.

The term "pharmaceutically acceptable carrier" refers to a substance that can be added to the active ingredient to help formulate or stabilize a drug without causing significantly harmful toxic effects to patients. The pharmaceutically acceptable carrier may include a pharmaceutically acceptable carrier commonly used in the formulation, and may include, but is not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like.

The pharmaceutical composition according to the present invention may further contain a lubricant, a wetting agent, a sweetener, a flavoring agent, an emulsifier, a suspension agent, a preservative, and the like, in addition to the ingredients described above. Suitable pharmaceutically acceptable carriers and formulations are described in detail in Remington's Pharmaceutical Science (19th ed., 1995) .

The pharmaceutical composition of the present invention may be administered parenterally, for example through intravenous injection, subcutaneous injection, intramuscular injection or intraperitoneal injection.

The suitable dose of the pharmaceutical composition according to the present invention may vary depending on factors such as the formulation method, administration method, and age, body weight, gender, pathological conditions, diet, administration time, administration route, excretion rate and responsiveness of the patient, and a general physician of ordinary skill can easily determine and prescribe a dose effective for the desired treatment or prevention. In a preferred embodiment, the present invention, the daily dose of the pharmaceutical composition according to the present invention may be within the range of 0.0001 to 100 mg/kg. The term "pharmaceutically effective amount" may mean an amount sufficient to prevent or treat cancer.

The pharmaceutical composition according to the present invention may be prepared into a unit dose form, or may be prepared by incorporation into a multi-dose container through formulation using a pharmaceutically acceptable carrier and/or excipient according to a method that can be easily implemented by those skilled in the art to which the present invention pertains. Here, the formulation may be in the form of a solution, a suspension or an emulsion in an oil or aqueous medium, or may be in the form of an extract, a powder, a suppository, a granule, a tablet, or a capsule. The composition may further contain a dispersant or a stabilizer.

In another aspect, the present invention is directed to a nucleic acid encoding the anti-c-Met antibody according to the invention. As used herein, the nucleic acid may be present in a cell or a cell lysate, or may be present in a partially purified form or in a substantially pure form. The nucleic acid may be "isolated" or "become substantially pure" when purified from other cellular components or other contaminants, for example, from nucleic acids or proteins of other cells, by standard techniques including, for example, alkaline/SDS treatment, CsCl banding, column chromatography, agarose gel electrophoresis and other methods well known in the art. The nucleic acid of the present invention may be, for example, DNA or RNA, and may or may not include an intron sequence.

According to the present invention, the nucleic acid encoding the anti-c-Met antibody may include a sequence selected from the group consisting of SEQ ID NOS: 55 to 69. Specifically, the polynucleotide sequence encoding the heavy chain of the antibody according to the present invention is represented by SEQ ID NOS: 55 or 57 to 63, and/or the polynucleotide sequence encoding the light chain of the antibody according to the present invention is represented by SEQ ID NOS: 56 or 64 to 69.

In another aspect, the present invention is directed to a recombinant expression vector including the nucleic acid. For expression of the anti-c-Met antibody or antigen-binding fragment thereof according to the present invention, DNA encoding partial- or full-length light or heavy chains may be prepared through standard molecular biology techniques (e.g., PCR amplification or cDNA cloning using a hybridoma expressing the desired antibody), and the DNA can be "operably linked" to a transcription and translation control sequence and inserted into an expression vector.

As used herein, the term "operably linked" may mean that the gene encoding the antibody is ligated into the vector such that the transcriptional and translational control sequences within the vector serve the intended function of regulating transcription and translation of the antibody gene. The expression vector and expression control sequences are selected so as to be compatible with the host cell used for expression. The light-chain gene of the antibody and the heavy-chain gene of the antibody are inserted into separate vectors, or both genes are inserted into the same expression vector. Antibodies are inserted into expression vectors by standard methods (e.g., ligation of complementary restriction enzyme sites on the antibody gene fragment and vector, or blunt-end ligation when no restriction enzyme site is present). In some cases, the recombinant expression vector can encode a signal peptide that facilitates secretion of the antibody chain from the host cell. The antibody-chain gene may be cloned into a vector such that the signal peptide is bound to the amino terminus of the antibody-chain gene in accordance with the frame. The signal peptide may be an immunoglobulin signal peptide or a heterologous signal peptide (i.e., a signal peptide derived from a protein other than immunoglobulin). In addition, the recombinant expression vector has a control sequence that controls the expression of the antibody-chain gene in the host cell. The term "control sequence" may include promoters, enhancers and other expression control elements (e.g., polyadenylation signals) that control the transcription or translation of the antibody-chain gene. Those skilled in the art will appreciate that the design of the expression vector can be varied by selecting different control sequences depending on factors such as selection of the host cell to be transformed and the level of expression of the protein.

In another aspect, the present invention is directed to a host cell including the nucleic acid or the vector. The host cell according to the present invention is preferably selected from the group consisting of animal cells, plant cells, yeast, *Escherichia coli* and insect cells, but is not limited thereto.

More specifically, the host cell according to the present invention may be a prokaryotic cell, such as *Escherichia coli, Bacillus subtilis, Streptomyces* sp., *Pseudomonas* sp., *Proteus mirabilis,* or *Staphylococcus* sp. The host cell may be a eukaryotic cell selected from fungi such as *Aspergillus* sp., yeast such as *Pichia pastoris, Saccharomyces cerevisiae, Schizosaccharomyces* sp., and *Neurospora crassa,* other lower eukaryotic cells, and higher eukaryotic cells such as cells derived from insects.

The host cell may also be derived from plants or mammals. Preferably, useful host cells may include, but are not limited to, monkey kidney cells (COS7), NSO cells, SP2/0 cells, Chinese hamster ovary (CHO) cells, W138, baby hamster kidney (BHK) cells, MDCK, myeloma cell lines, HuT 78 cells, HEK293 cells and the like. Particularly preferably, useful host cells are CHO cells.

The nucleic acid or the vector is transfected into a host cell. "Transfection" may be carried out using various techniques commonly used to introduce foreign nucleic acids (DNA or RNA) into prokaryotic or eukaryotic host cells, for example, electrophoresis, calcium phosphate precipitation, DEAE-dextran transfection or lipofection. Various expression host/vector combinations may be used to express the anti-c-met antibody according to the present invention. Suitable expression vectors for eukaryotic hosts include, for example, but are not limited to, expression control sequences derived from SV40, cow papillomavirus, adenovirus, adeno-associated virus, cytomegalovirus and retrovirus. Expression vectors that can be used for bacterial hosts include bacterial plasmids obtained from *Escherichia coli (E. coli),* such as pET, pRSET, pBluescript, pGEX2T, pUC vectors, col E1, pCR1, pBR322, pMB9 and derivatives thereof, plasmids having a wide host range such as RP4, phage DNA exemplified by a wide variety of phage lambda derivatives such as λgt10, λgt11 and NM989, and other DNA phages such as M13 and filamentous single-stranded DNA phages. Expression vectors useful for yeast cells include 2µ plasmids and derivatives thereof. A vector useful for insect cells is pVL 941.

In another aspect, the present invention is directed to a method for producing the anti-c-Met antibody or an antigen-binding fragment thereof according to the present invention, including culturing a host cell to express the anti-c-Met antibody or antigen-binding fragment thereof according to the present invention.

When a recombinant expression vector capable of expressing the anti-c-Met antibody or antigen-binding fragment thereof is introduced into a mammalian host cell, the antibody can be produced by culturing the host cell for a period of time sufficient to allow expression of the antibody in the host cell, more preferably, for a period of time sufficient to allow the antibody to be secreted into the culture medium.

In some cases, the expressed antibody may be separated from the host cell and purified to homogeneity. The separation or purification of the antibody can be carried out using separation and purification methods used for conventional proteins, for example, chromatography. Such chromatography may include, for example, affinity chromatography involving a protein A column or a protein G column, ion exchange chromatography, or hydrophobic chromatography. The antibody can be separated and purified by performing chromatography in combination with filtration, ultrafiltration, salting out, dialysis and the like.

The anti-c-Met antibody or antigen-binding fragment thereof and a pharmaceutical composition containing the same according to the present invention may be used in combination with a conventional therapeutic agent.

In another aspect, the present invention is directed to a co-administration composition for treating cancer containing the anti-c-Met antibody or antigen-binding fragment thereof along with an additional therapeutic agent for cancer, and a method of treating cancer using the same.

The other cancer therapeutic agents refer to all therapeutic agents other than the anti-c-Met antibody or antigen-binding fragment thereof according to the present invention that can be used for cancer treatment. According to the present invention, the therapeutic agent for cancer may be an immune checkpoint inhibitor, but is not limited thereto.

The human immune system has an immunity-screening system to inhibit hyperimmune reactions caused by overproliferation of T-cells. This immunity-screening system is called "immune checkpoint", and proteins involved in the immune checkpoint are called "immune checkpoint proteins". Essentially, the immune checkpoints perform the function of suppressing a hyperimmune response caused by overactivation and/or overproliferation of T-cells, but cancer cells prevent T-cells from attacking themselves and avoid the attack of the immune system by exploiting these immune checkpoints, ultimately resulting in progression of cancer.

It is already known in the art that diseases such as cancer can be treated using inhibitors of such immune checkpoints, and antibody drugs targeting immune checkpoint proteins are commercially available, and various immune checkpoint inhibitors are being developed.

The first therapeutic agent that was developed as an immune checkpoint inhibitor is ipilimumab, a monoclonal antibody specific to the immune checkpoint receptor CTLA-4 (cytotoxic T-lymphocyte associated antigen-4), which exhibited effects thereof in metastatic malignant melanoma. Subsequently, monoclonal antibodies specific for PD-1 (programmed cell death-1) and PD-L1 (programmed death ligand-1), a ligand for PD-1, are being developed, and typical examples thereof include nivolumab, pembrolizumab, avelumab, atezolizumab, durvalumab and the like. PD-1 or PD-L1 inhibitors exhibit effects in various tumors as well as in malignant melanoma.

According to the present invention, the immune checkpoint inhibitor means an immune checkpoint inhibitor or a checkpoint inhibitor, and may be an anti-CTLA-4 antibody, an anti-PD-1 antibody or an anti-PD-Ll antibody, but is not limited thereto. Specifically, examples of useful immune checkpoint inhibitors include, but are not limited to, ipilimumab, nivolumab, pembrolizumab, atezolizumab, avelumab and durvalumab.

According to the present invention, the cancer may be breast cancer, colon cancer, lung cancer, stomach cancer, liver cancer, blood cancer, bone cancer, pancreatic cancer, skin cancer, brain cancer, uterine cancer, nasopharyngeal cancer, laryngeal cancer, colon cancer, ovarian cancer, rectal cancer, colorectal cancer, vaginal cancer, small-intestine cancer, endocrine cancer, thyroid cancer, parathyroid cancer, ureteral cancer, urethral cancer, prostate cancer, bronchial cancer, bladder cancer, kidney cancer or bone-marrow cancer, but is not limited thereto.

The term "used in combination", "combined use" or "co-administration" means that the anti-c-Met antibody or antigen-binding fragment thereof and another additional therapeutic agent for cancer can be administered simultaneously, sequentially, or in reverse order, and can be administered in a combination of appropriate effective amounts that can be determined by those skilled in the art.

In one embodiment of the present invention, co-administration of the anti-PD-Ll antibody and the anti-c-Met antibody according to the present invention further inhibited tumor growth.

The composition for co-administration includes an anti-c-Met antibody, and the configurations associated therewith are the same as those included in the composition for preventing or treating cancer described above, so the description of each configuration applies equally to the composition for co-administration.

Hereinafter, the present invention will be described in more detail with reference to examples. However, it will be obvious to those skilled in the art that these examples are provided only for illustration of the present invention and should not be construed as limiting the scope of the present invention.

### Example 1: Construction of mutant library for affinity maturation

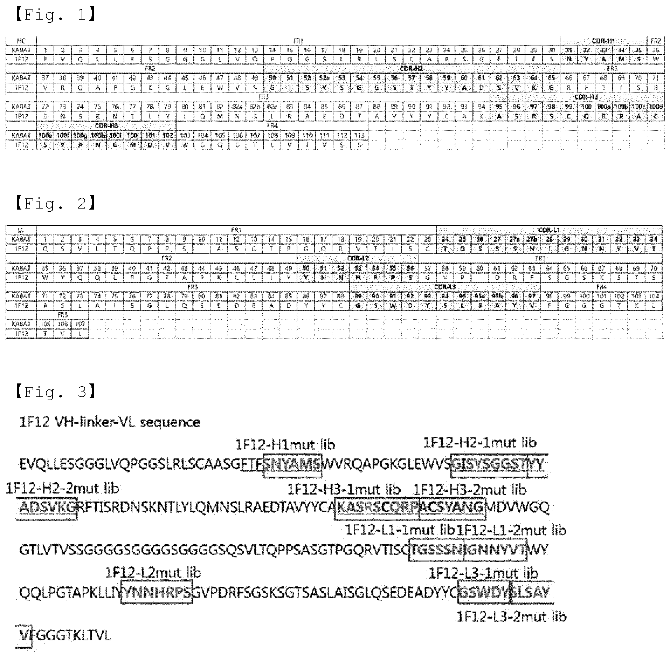

The c-Met target antibody 1F12 was selected using phage display technology, and directed evolution was used to improve the affinity of the antibody. The variable region of an antibody is divided into complementarity-determining regions (CDRs) and framework regions, and CDRs greatly contribute to antigen-antibody binding. The heavy-chain variable region and the light-chain variable region of the parent antibody are as shown in FIGS. 1 and 2. Based on the KABAT numbering method, the CDRs and framework regions of the antibody were divided (Table 1).

**[Table 1]**

| Heavyand light-chain CDR sequences for human/mouse c-Met cross-reacting scFv antibody | | | | |
|---|---|---|---|---|
| Antibody | C DR sequenc e | | | SEQ ID NO |
| 1F12 | Heavy Chain | CDRH1 | NYAMS | 1 |
| | | CDRH2 | GISYSGGSTYYADSVKG | 2 |
| | | CDRH3 | ASRSCQRP ACSY ANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |

10 kinds of mutant libraries (1F12-H1mut, 1F12-H2-1mut, 1F12-H2-2mut, 1F12-H3-1mut, 1F12-H3-2mut, 1F12-L1-1mut, 1F12-L1-2mut, 1F12-L2mut, 1F12-L3-1mut and 1F12-L3-2mut) were constructed in a total of 6 CDRs present in the variable regions of the heavy and light chains of the parent antibody (1F12) using an NNK degenerate codon (FIG. 3). The DNA sequence of the 1F12 mutant single-chain variable fragment (sfiI-VH-linker-VL-sfiI), in which the sequence of the CDR to be used for construction of mutant libraries was randomized, was obtained using overlapping extension PCR using the primers shown in Table 2 below.

**[Table 2]**

| Primer sequences | | |
|---|---|---|
| Primer | Sequence (5' -> 3') | SEQ ID NO |
| OPAL-F | CTACCGTGGCCCAGGCGGCCGAGGTGCAGCTGTTGG AGTC | 7 |
| OPAL-R | TGGTGCTGGCCGGCCTGGCCTAGGACCGTCAGCTTG GTG | 8 |
| 1F12H1mut-F | TGTGCAGCCTCTGGATTCACCTTTNNKNNKNNKNNK NNKNNKTGGGTCCGCCAGGCTCCAGGGAAG | 9 |
| 1F12H1mut-R | AAAGGTGAATCCAGAGGCTGCACA | 10 |
| 1F12H2-1mut-F | GGGAAGGGGCTGGAGTGGGTCTCANNKATCNNKNN KNNKNNKNNKNNKNNKTATTACGCTGATTCTGTAAA AGGT | 11 |
| 1F12H2-1mut-R | TGAGACCCACTCCAGCCCCTTCCC | 12 |
| 1F12H2-2mut-F | ATCTCTTATAGTGGTGGTAGTACANNKNNKNNKNNK NNKNNKNNKNNKCGGTTCACCATCTCCAGAGACAA T | 13 |
| 1F12H2-2mut-R | TGTACTACCACCACTATAAGAGAT | 14 |
| 1F12H3-1mut-F | GACACGGCCGTGTATTACTGTGCGARANNKNNKARA NNKTGTNNKNNKNNKGCTTGTTCTTATGCTAATGGT ATG | 15 |
| 1F12H3-1mut-R | CGCACAGTAATACACGGCCGTGTC | 16 |
| 1F12H3-2mut-F | GCTAGTCGTTCTTGTCAGCGGCCTNNKTGTNNKNNK NNKNNKNNKATGGACGTCTGGGGCCAGGGTACA | 17 |
| 1F12H3-2mut-R | AGGCCGCTGACAAGAACGACTAGC | 18 |
| 1F12L1-1mut-F | GGGCAGAGGGTCACCATCTCTTGTNNKNNKNNKNN KNNKNNKATTGGCAATAATTATGTCACCTGG | 19 |
| 1F12L1-1mut-R | ACAAGAGATGGTGACCCTCTGCCC | 20 |
| 1F12L1-2mut-F | TCTTGTACTGGCTCTTCATCTAATNNKNNKNNKNNK NNKNNKNNKTGGTACCAGCAGCTCCCAGGAACG | 21 |
| 1F12L1-2mut-R | ATTAGATGAAGAGCCAGTACAAGA | 22 |
| 1F12L2mut-F | ACGGCCCCCAAACTCCTCATCTATNNKNNKNNKNNK NNKNNKNNKGGGGTCCCTGACCGATTCTCTGGC | 23 |
| 1F12L2mut-R | ATAGATGAGGAGTTTGGGGGCCGT | 24 |
| 1F12L3-1mut-R | TGGTGCTGGCCGGCCTGGCCTAGGACCGTCAGCTTG GTGCCTCCGCCGAAGACATAAGCACTCAGGCTMNN MNNMNNMNNMNNACAGTAATAATCAGCCTCATCCT C | 25 |
| 1F12L3-2mut-R | TGGTGCTGGCCGGCCTGGCCTAGGACCGTCAGCTTG GTGCCTCCGCCGAAMNNMNNMNNMNNMNNMNNAT AATCCCAAGAACCACAGTAATA | 26 |

Here, cysteine is present in VH99 and VH100d of the heavy chain CDR-H3 (FIG. 1), and these two sites form an interchain disulfide bond to thereby stabilize a CDR-H3 structure. Therefore, a TGT codon, rather than an NNK codon, was used for these two sites in order to retain the residue. The pComb3X scFv expression vector (OmpA leader sequence-sfiI-VH-linker-VL-sfiI-His-HA-Amber codon-pIII) was linearized by removing the insert sequence using the sfiI (NEB) restriction enzyme. In this linearized vector, sfiI-VH-linker-VL-sfiI, in which the positions of respective CDRs are randomized into 20 amino acids by an NNK degenerate codon, was inserted into the linearized vector using T4 ligase (NEB) to construct mutant libraries.

### Example 2: Selection of antibody with improved affinity

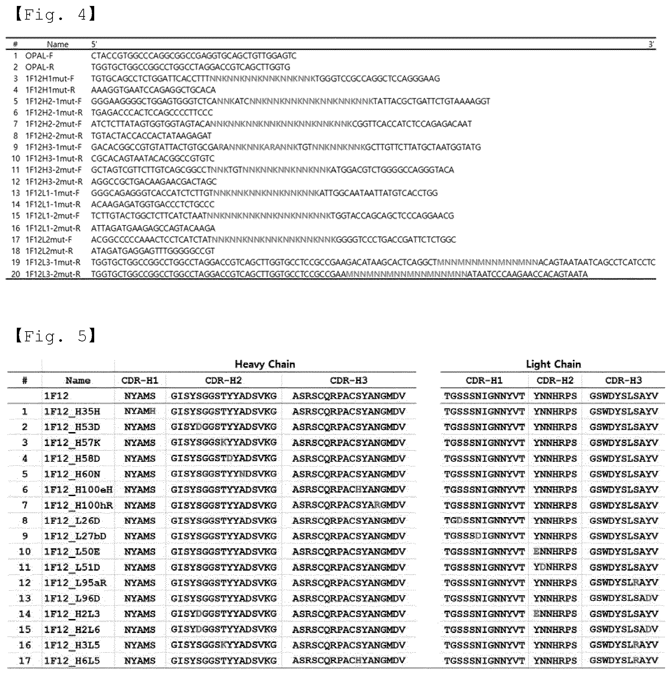

The constructed mutant libraries used TG1 *E. coli* as a host cell, and had about 3.10 x 10¹⁰ transformants. These mutant libraries were recovered in phage form, and antibody pools exhibiting higher binding ability to c-Met were enriched by applying phage display technology, and affinity variants were selected through screening using ELISA. 13 clones with improved affinity were selected at first, and the clones were 1F12_H35H, 1F12_H53D, 1F12_H57K, 1F12_H58D, 1F12_H60N, 1F12_H100eH, 1F12_H100hR 1F12_L26D, 1F12_L27bD, 1F12_L50E, 1F12_L51D, 1F12_L95aR and 1F12_L96D. 1F12-H35H means a mutation in which the amino acid at position 35 of the 1F12 heavy chain is substituted with histidine (H) based on KABAT numbering, and 1F12_L26D means a mutation in which the amino acid at position 26 of the 1F12 light chain is substituted with aspartic acid. For these 13 affinity-improving mutant antibodies, full-length human IgG1 production vectors were constructed in mammalian cells, and this was performed according to the manufacturer's manual using the Expi293 expression system (Gibco). As a result, 1F12_H100hR (a clone in which the amino acid at the position 100h in the heavy chain in the parent antibody was substituted with arginine) did not express all antibodies during repeated preparation. Six heavy-chain CDR residue-substituted antibodies and six light-chain CDR residue-substituted antibodies of 1F12 were obtained, and four additional antibodies (1F12_H2L3, 1F12_H2L6, 1F12_H3L5, 1F12_H6L5) were produced by combining these antibodies. 1F12_H2L3 is an antibody produced by combining a heavy-chain expression vector, in which the amino acid at position 53 of the heavy-chain variable region is substituted with aspartic acid, with a light-chain expression vector, in which the amino acid at position 50 of the light-chain variable region is substituted with glutamic acid (meaning that it was produced with the second and third expression vectors among 7 types of heavy-chain mutant expression vectors and 6 types of light-chain mutant expression vectors, respectively). In conclusion, antibodies other than 1F12_H100hR in FIG. 5 were expressed and purified in mammalian cells and used in subsequent experiments.

The CDR sequences of a total of 17 types of improved antibodies are shown in Table 3, and the heavy-chain and light-chain variable-region sequences are shown in Table 4. In addition, the polynucleotide sequence encoding each improved antibody is shown in Table 5.

### [TABLE 3]

Amino acid sequences of heavy-chain CDRs and light-chain CDRs

| Antibody | CDR sequence | | | SEQ ID NO |
|---|---|---|---|---|
| 1F12_H35 H | Heavy Chain | CDRH1 | NYAMH | 27 |
| | | CDRH2 | GISYSGGSTYYADSVKG | 2 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H53 D | Heavy Chain | CDRH1 | NYAMS | 1 |
| | | CDRH2 | GISYDGGSTYYADSVKG | 28 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H57 K | Heavy Chain | CDRH1 | NYAMS | 1 |
| | | CDRH2 | GISYSGGSKYYADSVKG | 29 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H58 D | Heavy Chain | CDRH1 | NYAMS | 1 |
| | | CDRH2 | GISYSGGSTDYADSVKG | 30 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H60 N | Heavy Chain | CDRH1 | NYAMS | 1 |
| | | CDRH2 | GISYSGGSTYYNDSVKG | 31 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H10 0eH | Heavy Chain | CDRH1 | NYAMS | 1 |
| | | CDRH2 | GISYSGGSTYYADSVKG | 2 |
| | | CDRH3 | ASRSCQRPACHYANGMDV | 32 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_H10 0hR | Heavy Chain | CDRH1 | NYAMS | 1 |
| | | CDRH2 | GISYSGGSTYYADSVKG | 2 |
| | | CDRH3 | ASRSCQRPACSYARGMDV | 33 |
| | Light Chain | CDRL1 | TGSSSNIGNNYVT | 4 |
| | | CDRL2 | YNNHRPS | 5 |
| | | CDRL3 | GSWDYSLSAYV | 6 |
| 1F12_L26 D | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGDSSNIGNNYVT | 34 |
| | | CDRL2 | YNNHRPS | 5 |
| | | CDRL3 | GSWDYSLSAYV | 6 |
| 1F12_L27b D | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGSSSDIGNNYVT | 35 |
| | | CDRL2 | YNNHRPS | 5 |
| | | CDRL3 | GSWDYSLSAYV | 6 |
| 1F12_L50 E | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGSSSNIGNNYVT | 4 |
| | | CDRL2 | ENNHRPS | 36 |
| | | CDRL3 | GSWDYSLSAYV | 6 |
| 1F12_L51 D | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGSSSNIGNNYVT | 4 |
| | | CDRL2 | YDNHRPS | 37 |
| | | CDRL3 | GSWDYSLSAYV | 6 |
| 1F12_L95a R | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLRAYV | 38 |
| IF12_L96 D | Heavy Chain | CDRH1 | NYAMS | 1 |
| | | CDRH2 | GISYSGGSTYYADSVKG | 2 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSADV | 39 |
| 1F12_H2L 3 | Heavy Chain | CDRH1 | NYAMS | 1 |
| | | CDRH2 | GISYDGGSTYYADSVKG | 28 |
| | | CDRH3 | ASRSCQRPACSYANGMDV | 3 |
| | Light Chain | CDRL1 | TGSSSNIGNNYVT | 4 |
| | | CDRL2 | ENNHRPS | 36 |
| | | CDRL3 | GSWDYSLSAYV | 6 |
| 1F12_H2L 6 | Heavy Chain | CDRH1 | NYAMS | 1 |
| | | CDRH2 | GISYDGGSTYYADSVKG | 28 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLSADV | 39 |
| 1F12_H3L 5 | Heavy Chain | CDRH1 | NYAMS | 1 |
| | | CDRH2 | GISYSGGSKYYADSVKG | 29 |
| | | CDRH3 | ASRSCQRPACSYANGMDV | 3 |
| | Light Chain | CDRL1 | TGSSSNIGNNYVT | 4 |
| | | CDRL2 | YNNHRPS | 5 |
| | | CDRL3 | GSWDYSLRAYV | 38 |
| 1F12_H6L 5 | Heavy Chain | CDRH1 | NYAMS | 1 |
| | | CDRH2 | GISYSGGSTYYADSVKG | 2 |
| | | CDRH3 | ASRSCQRPACHYANGMDV | 32 |
| | Light Chain | CDRL1 | TGSSSNIGNNYVT | 4 |
| | | CDRL2 | YNNHRPS | 5 |
| | | CDRL3 | GSWDYSLRAYV | 38 |

**[TABLE 4]**

| Heavyand light-chain variable-region sequences | | | |
|---|---|---|---|
| Antibody | Variable-region sequence | | SEQ ID NO |
| 1F12_H35H | Heavy chain | EVQLLESGGGLVQPGGSLRLSCAASGFTFSNY AMHWVRQAPGKGLEWVSGISYSGGSTYYADS VKGRFTISRDNSKNTLYLQMNSLRAEDTAVY YCAKASRSCQRPACSYANGMDVWGQGTLVT VSS | 40 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H53D | Heavy chain | EVQLLESGGGLVQPGGSLRLSCAASGFTFSNY AMSWVRQAPGKGLEWVSGISYDGGSTYYADS VKGRFTISRDNSKNTLYLQMNSLRAEDTAVY YCAKASRSCQRPACSYANGMDVWGQGTLVT | 42 |
| | | VSS | |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H57K | Heavy chain | EVQLLESGGGLVQPGGSLRLSCAASGFTFSNY AMSWVRQAPGKGLEWVSGISYSGGSKYYADS VKGRFTISRDNSKNTLYLQMNSLRAEDTAVY YCAKASRSCQRPACSYANGMDVWGQGTLVT VSS | 43 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H58D | Heavy chain | EVQLLESGGGLVQPGGSLRLSCAASGFTFSNY AMSWVRQAPGKGLEWVSGISYSGGSTDYADS VKGRFTISRDNSKNTLYLQMNSLRAEDTAVY YCAKASRSCQRPACSYANGMDVWGQGTLVT VSS | 44 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H60N | Heavy chain | EVQLLESGGGLVQPGGSLRLSCAASGFTFSNY AMSWVRQAPGKGLEWVSGISYSGGSTYYNDS VKGRFTISRDNSKNTLYLQMNSLRAEDTAVY YCAKASRSCQRPACSYANGMDVWGQGTLVT VSS | 45 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H100eH | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACHYANGMDVWGQGTLVT VSS | 46 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_H100hR | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYARGMDVWGQGTLVT | 47 |
| | | VSS | |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS AYVFGGGTKLTVL | 41 |
| 1F12_L26D | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT VSS | 48 |
| | Light chain | QSVLTQPPSASGTPGQRVTISCTGDSSNIGNNY VTWYQQLPGTAPKLLIYYNNHRPSGVPDRFSG SKSGTSASLAISGLQSEDEADYYCGSWDYSLS AYVFGGGTKLTVL | 49 |
| 1F12_L27bD | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT VSS | 48 |
| | Light chain | QSVLTQPPSASGTPGQRVTISCTGSSSDIGNNY VTWYQQLPGTAPKLLIYYNNHRPSGVPDRFSG SKSGTSASLAISGLQSEDEADYYCGSWDYSLS AYVFGGGTKLTVL | 50 |
| 1F12_L50E | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT VSS | 48 |
| | Light chain | QSVLTQPPSASGTPGQRVTISCTGSSSNIGNNY VTWYQQLPGTAPKLLIYENNHRPSGVPDRFSG SKSGTSASLAISGLQSEDEADYYCGSWDYSLS AYVFGGGTKLTVL | 51 |
| 1F12_L51D | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT VSS | 48 |
| | Light chain | QSVLTQPPSASGTPGQRVTISCTGSSSNIGNNY VTWYQQLPGTAPKLLIYYDNHRPSGVPDRFSG SKSGTSASLAISGLQSEDEADYYCGSWDYSLS AYVFGGGTKLTVL | 52 |
| 1F12_L95aR | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT | 48 |
| | | VSS | |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLR AYVFGGGTKLTVL | 53 |
| 1F12_L96D | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACSYANGMDVWGQGTLVT VSS | 48 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS ADVFGGGTKLTVL | 54 |
| 1F12_H2L3 | Heavy chain | EVQLLESGGGLVQPGGSLRLSCAASGFTFSNY AMSWVRQAPGKGLEWVSGISYDGGSTYYADS VKGRFTISRDNSKNTLYLQMNSLRAEDTAVY YCAKASRSCQRPACSYANGMDVWGQGTLVT VSS | 42 |
| | Light chain | QSVLTQPPSASGTPGQRVTISCTGSSSNIGNNY VTWYQQLPGTAPKLLIYENNHRPSGVPDRFSG SKSGTSASLAISGLQSEDEADYYCGSWDYSLS AYVFGGGTKLTVL | 51 |
| 1F12_H2L6 | Heavy chain | EVQLLESGGGLVQPGGSLRLSCAASGFTFSNY AMSWVRQAPGKGLEWVSGISYDGGSTYYADS VKGRFTISRDNSKNTLYLQMNSLRAEDTAVY YCAKASRSCQRPACSYANGMDVWGQGTLVT VSS | 42 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLS ADVFGGGTKLTVL | 54 |
| 1F12_H3L5 | Heavy chain | EVQLLESGGGLVQPGGSLRLSCAASGFTFSNY AMSWVRQAPGKGLEWVSGISYSGGSKYYADS VKGRFTISRDNSKNTLYLQMNSLRAEDTAVY YCAKASRSCQRPACSYANGMDVWGQGTLVT VSS | 43 |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLR AYVFGGGTKLTVL | 53 |
| 1F12_H6L5 | Heavy chain | EVQLLESGGGLVQPGGSLRLSCAASGFTFSNY AMSWVRQAPGKGLEWVSGISYSGGSTYYADS VKGRFTISRDNSKNTLYLQMNSLRAEDTAVY YCAKASRSCQRPACHYANGMDVWGQGTLVT | 46 |
| | | VSS | |
| | Light chain | QSVLTQPPSASGTPGQRVTISCTGSSSNIGNNY VTWYQQLPGTAPKLLIYYNNHRPSGVPDRFSG SKSGTSASLAISGLQSEDEADYYCGSWDYSLR AYVFGGGTKLTVL | 53 |

**[TABLE 5]**

| Polynucleotide sequences used for encoding of heavyand light-chain variable regions | | | |
|---|---|---|---|
| Antibody | Polynucleotide sequence | | SEQ ID NO |
| 1F12_H35H | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGCACTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 55 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT | 56 |
| | | CGGCGGAGGCACCAAGCTTACGGTCCTA | |
| 1F12_H53D | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATGATGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 57 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_H57K | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTAAATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 58 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_H58D | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA | 59 |
| | | TGAGCTGGGTCCGCCAGGCTCCAGGGAAGGGGC TGGAGTGGGTCTCAGGGATCTCTTATAGTGGTG GTAGTACAGATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_H60N | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACAATGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 60 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_H100eH | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG | 61 |
| | | AGGACACGGCCGTGTATTACTGTGCGAAAGCTA GTCGTTCTTGTCAGCGGCCTGCTTGTCATTATGC TAATGGTATGGACGTCTGGGGCCAGGGTACACT GGTCACCGTGAGCTCA | |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_H100hR | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAGAGGTATGGACGTCTGGGGCCAGGGTACACT GGTCACCGTGAGCTCA | 62 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 56 |
| 1F12_L26D | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCGATTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 64 |
| 1F12_L27bD | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCTCTTCATCTGATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 65 |
| 1F12_L50E | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATGAAAATAATCATCGGCC | 66 |
| | | AAGCGGGGTCCCTGACCGATTCTCTGGCTCCAA GTCTGGCACCTCAGCCTCCCTGGCCATCAGTGG GCTCCAGTCCGAGGATGAGGCTGATTATTACTG TGGTTCTTGGGATTATAGCCTGAGTGCTTATGTC TTCGGCGGAGGCACCAAGCTTACGGTCCTA | |
| 1F12_L51D | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATGATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTTATGTCTT CGGCGGAGGCACCAAGCTTACGGTCCTA | 67 |
| 1F12_L95aR | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGAGCTTATGTCTT | 68 |
| | | CGGCGGAGGCACCAAGCTTACGGTCCTA | |
| 1F12_L96D | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 63 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTGATGTCTT CGGCGGAGGCACCAAGCTTACGGTCCTA | 69 |
| 1F12_H2L3 | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATGATGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 57 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATGAAAATAATCATCGGCC AAGCGGGGTCCCTGACCGATTCTCTGGCTCCAA GTCTGGCACCTCAGCCTCCCTGGCCATCAGTGG GCTCCAGTCCGAGGATGAGGCTGATTATTACTG TGGTTCTTGGGATTATAGCCTGAGTGCTTATGTC TTCGGCGGAGGCACCAAGCTTACGGTCCTA | 66 |
| 1F12_H2L6 | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA | 57 |
| | | TGAGCTGGGTCCGCCAGGCTCCAGGGAAGGGGC TGGAGTGGGTCTCAGGGATCTCTTATGATGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGTGCTGATGTCTT CGGCGGAGGCACCAAGCTTACGGTCCTA | 69 |
| 1F12_H3L5 | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTAAATATTACGCTGATTCTGTAAAAGGTC GGTTCACCATCTCCAGAGACAATTCCAAGAACA CGCTGTATCTGCAAATGAACAGCCTGAGAGCCG AGGACACGGCCGTGTATTACTGTGCGAAAGCTA GTCGTTCTTGTCAGCGGCCTGCTTGTTCTTATGC TAATGGTATGGACGTCTGGGGCCAGGGTACACT GGTCACCGTGAGCTCA | 58 |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGAGCTTATGTCTT CGGCGGAGGCACCAAGCTTACGGTCCTA | 68 |
| 1F12_H6L5 | Heavy chain | GAGGTGCAGCTGTTGGAGTCTGGGGGAGGCTTG GTACAGCCTGGGGGGTCCCTGAGACTCTCCTGT GCAGCCTCTGGATTCACCTTTAGCAATTATGCTA TGAGCTGGGTCCGCCAGGCTCCAGGGAAGGGGC TGGAGTGGGTCTCAGGGATCTCTTATAGTGGTG GTAGTACATATTACGCTGATTCTGTAAAAGGTC GGTTCACCATCTCCAGAGACAATTCCAAGAACA CGCTGTATCTGCAAATGAACAGCCTGAGAGCCG | 61 |
| | | AGGACACGGCCGTGTATTACTGTGCGAAAGCTA GTCGTTCTTGTCAGCGGCCTGCTTGTCATTATGC TAATGGTATGGACGTCTGGGGCCAGGGTACACT GGTCACCGTGAGCTCA | |
| | Light chain | CAGTCTGTGCTGACTCAGCCACCCTCAGCGTCTG GGACCCCCGGGCAGAGGGTCACCATCTCTTGTA CTGGCTCTTCATCTAATATTGGCAATAATTATGT CACCTGGTACCAGCAGCTCCCAGGAACGGCCCC CAAACTCCTCATCTATTATAATAATCATCGGCCA AGCGGGGTCCCTGACCGATTCTCTGGCTCCAAG TCTGGCACCTCAGCCTCCCTGGCCATCAGTGGGC TCCAGTCCGAGGATGAGGCTGATTATTACTGTG GTTCTTGGGATTATAGCCTGAGAGCTTATGTCTT CGGCGGAGGCACCAAGCTTACGGTCCTA | 68 |

### Example 3: Analysis of agonist activity

Analysis of agonist activity is essential for the development of c-Met antibodies. General antibodies have a bivalent structure with two paratopes that recognize targets in a Y-form. For this reason, one c-Met antibody binds to two c-Met, target antigens, which induce c-Met dimerization, resulting in agonist activity that activates the downstream signaling pathway. Genentech developed a 5D5 antibody using hybridoma technology, but this antibody binds to c-Met and exhibits a similar effect to HGF/SF, a ligand of c-Met, resulting in agonist activity that improves the signal transduction pathway. In order to minimize this phenomenon, the 5D5 antibody was improved as OA-5D5, a one-arm form, and agonist activity was minimized.

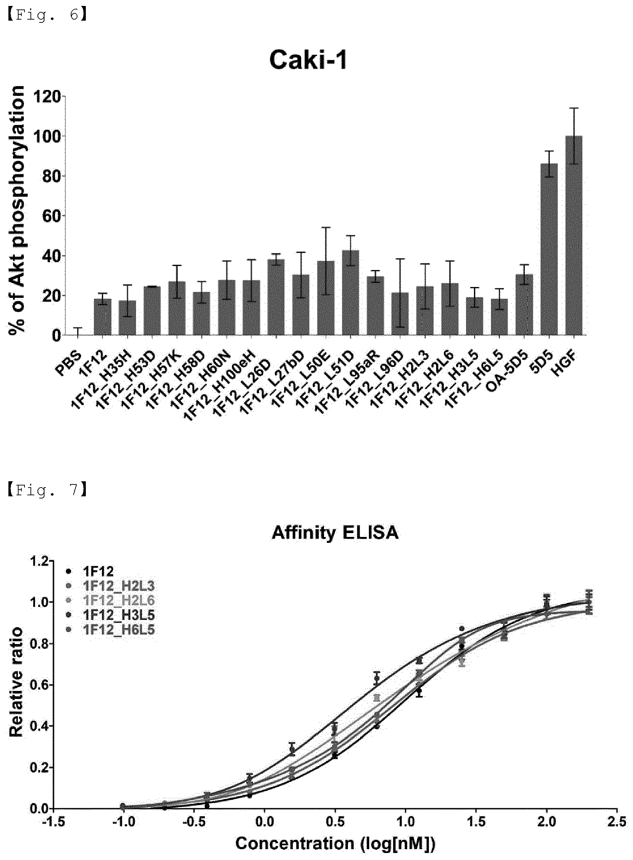

The degree of Akt phosphorylation was measured in order to quantify the agonist activity of the parent antibody 1F12 and 16 affinity variants. Specifically, when the Caki-1 renal cell carcinoma cell line in a 96-well cell culture plate reached about 70% confluency per well in RPMI1640 complete medium (+10% FBS), serum starvation was conducted for 24 hours. The cell line was treated with the parent antibody and 16 affinity variants, and c-Met agonist antibody 5D5, OA-5D5 exhibiting minimal agonist activity, and c-Met ligand HGF/SF (R&D systems) were used as control groups. PBS was treated in the same volume as the sample treated with the vehicle of the sample, antibodies were treated in 10 µg/mL, and HGF/SF was treated in 50 ng/L. The treatment time for the antibody and ligand was 30 minutes, and the experiment was performed in triplicate. Washing was performed once with 1X PBS immediately 30 minutes after treatment with each sample, and cell lysis was performed using a lysis buffer. Then, Akt phosphorylation was measured according to the manufacturer's manual using a PathScan® Phospho-Akt Sandwich ELISA Kit (Cell Signaling Technology), the luminescent signal for each well was measured, the value for the PBS treatment group was converted to 0% and the value for the HGF/SF treatment group was converted to 100%, and the agonist activity of each antibody was digitized. The 5D5, c-Met agonist antibody induced 86.01% of Akt phosphorylation and exhibited agonist activity similar to that of HGF. The OA-5D5 (one-armed monovalent antibody) modified to minimize agonist activity exhibited decreased agonist activity of 30.46%. In comparison, the agonist activity of the 1F12 parent antibody was found to be 18.23%, and the agonist activity of the 16 types of affinity variants are shown in Table 6 below (FIG. 6).

**[Table 6]**

| Analysis of agonist activity of antibodies in Caki-1 cell line | | |
|---|---|---|
| | Mean (%) | Standard deviation |
| PBS | 0.00 | 3.12 |
| 1F12 | 18.23 | 2.31 |
| 1F12_H35H | 17.32 | 6.51 |
| 1F12_H53D | 24.47 | 0.23 |
| 1F12_H57K | 26.84 | 6.77 |
| 1F12_H58D | 21.53 | 4.43 |
| 1F12_H60N | 27.70 | 7.94 |
| 1F12_H100eH | 27.46 | 8.59 |
| 1F12_L26D | 38.06 | 2.31 |
| 1F12_L27bD | 30.27 | 9.37 |
| 1F12_L50E | 37.17 | 13.70 |
| 1F12_L51D | 42.49 | 6.12 |
| 1F12_L95aR | 29.50 | 2.38 |
| 1F12_L96D | 21.20 | 14.10 |
| 1F12_H2L3 | 24.53 | 9.29 |
| 1F12_H2L6 | 25.97 | 9.34 |
| 1F12_H3L5 | 18.92 | 4.05 |
| 1F12_H6L5 | 18.18 | 4.29 |
| OA-5D5 | 30.46 | 4.10 |
| 5D5 | 86.01 | 5.36 |
| HGF | 100.00 | 11.41 |

### Example 4: Affinity analysis

ELISA-based affinity analysis was performed in triplicate. Recombinant human c-Met (Sino biological) was coated at 50 ng/well on a 96-well ELISA plate (Costar) overnight at 4°C. The next day, blocking was performed with a 3% skim milk solution for 1 hour, and then washing was performed three times using 1X PBST (Cell Signaling Technology). Each antibody was diluted by 1/2 from 200 nM in PBS (Gibco), treated in a volume of 100 µL in each well, and allowed to stand at room temperature for 1 hour. After washing three times using 1XPBST (Cell Signaling Technology), anti-human Fab-HRP (Thermo Scientific) secondary antibody diluted in 3% skim milk solution at 1:10000 was added in an amount of 100 µL to each well and then allowed to stand at room temperature for 1 hour. After washing each well 5 times using 1X PBST (Cell Signaling Technology), each well was treated with 100 µL of TMB solution (Thermo Scientific) and was then treated with 100 µL of a stop solution (Cell Signaling Technology) to terminate the reaction when the color began to turn blue. OD₄₅₀ was measured using a UV/VIS spectrophotometer, and this value was normalized. The result showed that the affinity of the 1F12_H2L3, 1F12_H2L6, 1F12_H3L5 and 1F12_H6L5 clones was increased compared to the parent antibody (1F12), and the 1F12_H3L5 clone exhibited the highest affinity (FIG. 7).

### Example 5: Analysis of inhibitory ability against cancer cell growth

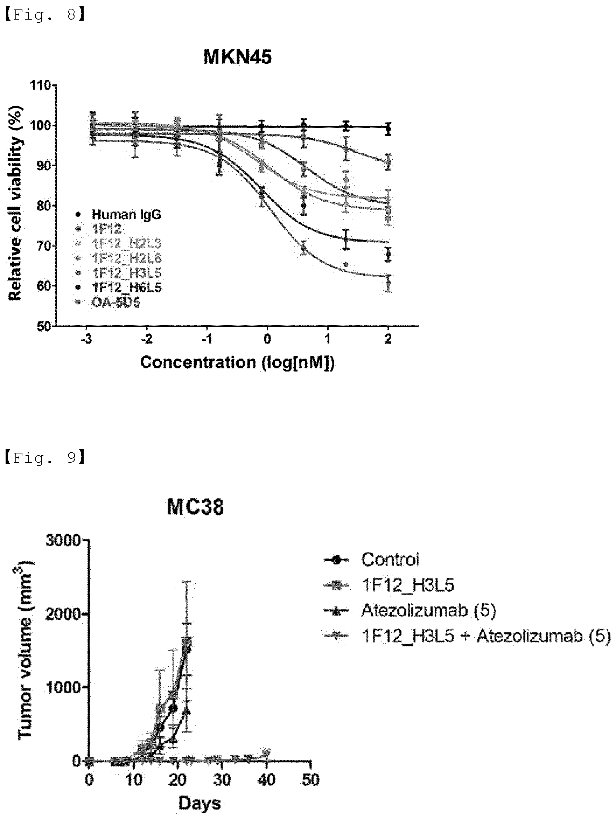

MKN45 is a c-Met-amplified gastric cancer cell line, obtained from a JCRB Cell Bank (Japan), and cultured and then maintained in a RPMI 1640 medium supplemented with 10% FBS. Onartuzumab (OA-5D5; monovalent c-Met Antibody, Genentech) was used as a control antibody, and an antibody expression vector was constructed based on the published antibody sequence. Transient expression was performed using the Expi293 expression system (Gibco), purification was performed through affinity chromatography using MabSelect SuRe (GE) mounted on an AKTA avant (GE), and purity of 98% or more was detected through SE-HPLC analysis. Similarity to results reported in the literature was determined through ELISA and SPR analysis. For the analysis of inhibitory activity against cell growth, the cells were seeded at a density of 3,000 cells/well in a complete culture medium containing RPMN 1640 and 10% FBS on a 96-well assay plate (Corning, 3610) and incubated overnight to attach the cells thereto, the medium was removed, and the antibody was diluted in a complete culture medium by 1/5 from 100 nM at maximum and treated with 100 µL of the medium. After 72 hours, each well was treated with 100 µL of Cell Titer Glo (Promega), and cell viability was analyzed using an Infinite M200 Pro (TECAN). The result showed that 1F12_H2L3, 1F12_H2L6, 1F12_H3L5 and 1F12_H6L5 antibodies exhibited better efficacy than the non-modified (parent) antibody (1F12) and the control antibody, onartuzumab, and the 1F12_H3L5 antibody was determined to exhibit the best efficacy (FIG. 8).

### Example 6: Evaluation of tumor growth inhibitory ability in animal models

In order to evaluate tumor growth inhibitory ability in a tumor-cell-transplanted animal model, MC38 cells, which are mouse colon cancer tumor cells, were transplanted into mice to construct a tumor animal model, and the tumor growth inhibitory ability was evaluated upon administration of 1F12_H3L5.

MC38 was prepared at a density of 200,000 cells/100 µL. At this time, a solution containing Hank's Salt (HBSS) solution (Gibco) and Basal Matrigel (Corning®) mixed in 1:1 was used. The prepared cells were transplanted at 100 µL into the rear part of the right side of the back of 7- to 8-week-old female C57BL/6 mice using a 1cc syringe (26G).

The administration of 1F12_H3L5 (20 mg/kg, i.p.) and atezolizumab (5 mg/kg, i.p.) was started from the 5^{th} day after transplantation of the MC38 cell line into each mouse, and was conducted twice a week. In order to evaluate reactivity upon co-administration, 1F12_H3L5 (20 mg/kg, i.p.) and atezolizumab (5 mg/kg, i.p.) were administered like a single administration group on the 5^{th} day after tumor transplantation. 1F12_H3L5 (20 mg/kg, i.p.) was administered first, and then atezolizumab (5 mg/kg, i.p.) was administered. The tumor size was calculated by measuring the long axis and short axis in millimeter (mm) units using calipers and applying the measured values to the calculation formula of [(long axis) × (short axis)² × 0.5].

The result of evaluation showed that the MC38 tumor animal model exhibited no 1F12_H3L5 (20 mg/kg, i.p.), and the atezolizumab (5 mg/kg, i.p.) administration group exhibited a tumor inhibition of about 40 to about 50%. The co-administration group (1F12_H3L5 + atezolizumab) in the same model inhibited tumor formation compared to the control group (22 days after tumor transplantation), and tumors were formed in only one subject in the co-administration group and tumors were not still formed in the remaining four subjects (at about 43 days after the experiment was terminated).

The results of animal tests conducted in the MC38 tumor animal model suggest that the therapeutic efficacy of 1F12_H3L5 can be improved through co-administration of 1F12_H3L5 and atezolizumab (PD-L1 inhibitor), which is known as a typical immune checkpoint inhibitor (FIG. 9).

### Example 7: Evaluation of responsiveness to combination therapy with radiation therapy in animal models

In order to evaluate the tumor growth inhibitory ability in a tumor-cell-transplanted animal model, MC38 cells, which are mouse colon cancer tumor cells, were transplanted into mice to create a tumor animal model, and the tumor growth inhibitory ability was evaluated upon the administration of 1F12_H3L5.

MC38 was prepared at a density of 200,000 cells/100 µL. At this time, a solution containing Hank's Salt (HBSS) solution (Gibco) and Basal Matrigel (Corning®) mixed at a ratio of 1:1 was used. The prepared cells were transplanted in an amount of 100 µL into the rear part of the right side of the back of 7- to 8-week-old female C57BL/6 mice using a 1cc syringe (26G). The tumor size was calculated by measuring the long axis and short axis in millimeter (mm) units using calipers and applying the measured values to the calculation formula of [(long axis) × (short axis)² × 0.5]. For radiotherapy, mice were anesthetized 20 days after tumor implantation, and the subcutaneous- implanted tumor was irradiated with a single dose of 2Gy using a radiation irradiator.

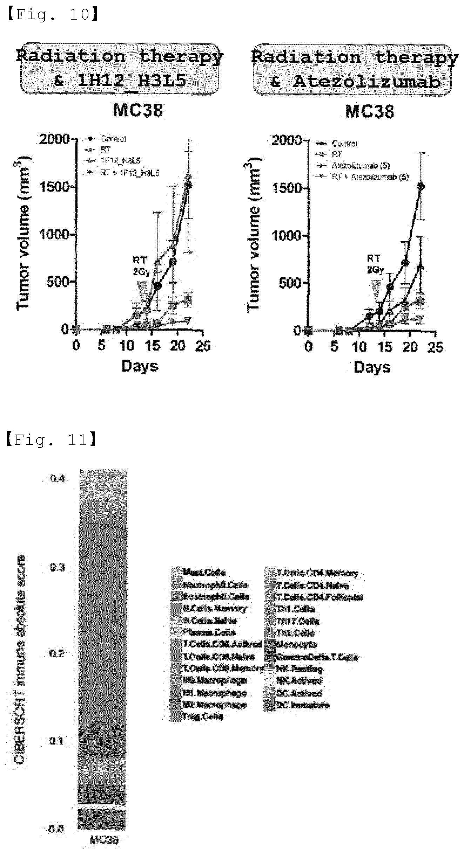

The result of the test showed that approximately 80% of tumor growth inhibition was observed in the irradiated group at the end of the test, and when 1F12_H3L5 or atezolizumab was administered along with irradiation, a tumor growth inhibitory ability of about 90% was found (FIG. 10) .

### Example 8: Analysis of immune cell expression in MC38 animal model tumor

In order to analyze mechanisms such as strategies for co-administration with an immune checkpoint inhibitor or the like, an MC38 animal model was constructed, and the expression of immune cells in tumor tissues was analyzed.

MC38 was prepared at 200,000 cells/100 µL. At this time, a solution containing Hank's Salt (HBSS) solution (Gibco) and Basal Matrigel (Corning®) mixed at a ratio of 1:1 was used. The prepared cells were transplanted in an amount of 100 µL into the rear part of the right side of the back of 7- to 8-week-old female C57BL/6 mice using a 1cc syringe (26G). When the tumor size reached about 800-1,000 mm³, tumor tissue was obtained by autopsy, and analysis was performed by RNA sequencing using the separated tumor tissue. The analysis was conducted using gene expression signatures for each major immune cell previously reported in the literature.

The result of detection of the expression of major immune cells in the MC38 tumor tissue showed that a lot of immune cells were infiltrated in tumors (FIG. 11). This suggests that responsiveness to the immune checkpoint inhibitor treatment and tumor-immunity-related treatment are highly likely to appear in the MC38 tumor model, and it is expected that treatment responsiveness can be predicted depending on the tendency of immune cell expression in tumors in subsequent tests.

### Example 9: Analysis of immune checkpoint PD-L1 in tumors

In order to detect a change in the expression of PD-L1, an immune checkpoint, in the MC38 tumor cells of the mice administered with 1F12_H3L5 and the mice not administered with 1F12_H3L5, the tumor tissue was separated into single cells. Analysis of PD-L1 in tumor cells was performed by staining the individual cells with CD45 and PD-L1, which are immune cell markers. In order to exclude immune cells invading the tumor cells, the expression of PD-L1 was detected in cells not expressing CD45.

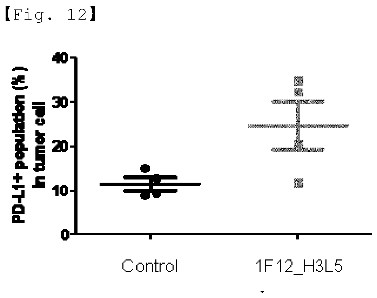

The result showed that the number of cells expressing PD-L1 increased more than two times in mouse subjects administered with 1F12_H3L5 (FIG. 12). This suggests that sensitivity to 1F12_H3L5 and PD-L1 inhibitors may be increased.

### [Industrial Applicability]

The antibody or antigen-binding fragment thereof that binds to c-Met according to the present invention can bind to human and mouse c-Met with high affinity and thus exhibit more accurate preclinical results in the efficacy evaluation using mouse tumor models. The antibody or antigen-binding fragment thereof that binds to c-Met according to the present invention can be useful for the prevention or treatment of target cancer.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence List Free Text]

An electronic file was attached.

## Claims

1. An anti-c-Met antibody or antigen-binding fragment thereof comprising:
a heavy-chain variable region comprising a heavy chain CDR1 having an amino acid sequence of SEQ ID NO: 1 or 27, a heavy chain CDR2 having an amino acid sequence selected from the group consisting of SEQ ID NOS: 2 and 28 to 31, and a heavy chain CDR3 having an amino acid sequence selected from the group consisting of SEQ ID NOS: 3, 32 and 33; and
a light-chain variable region comprising a light chain CDR1 having an amino acid sequence selected from the group consisting of SEQ ID NOS: 4, 34 and 35, a light chain CDR2 having an amino acid sequence selected from the group consisting of SEQ ID NOS: 5, 36 and 37, and a light chain CDR3 having an amino acid sequence selected from the group consisting of SEQ ID NOS: 6, 38 and 39.

2. The anti-c-Met antibody or antigen-binding fragment thereof according to claim 1, wherein the anti-c-Met antibody or antigen-binding fragment thereof comprises a heavy-chain variable region including an amino acid sequence selected from the group consisting of SEQ ID NOS: 40 and 42 to 48, and a light-chain variable region selected from the group consisting of SEQ ID NOS: 41 and 49 to 54.

3. The anti-c-Met antibody or antigen-binding fragment thereof according to claim 1 or 2, wherein the anti-c-Met antibody is a monoclonal antibody.

4. The anti-c-Met antibody or antigen-binding fragment thereof according to claim 1 or 2, wherein the antigen-binding fragment is selected from the group consisting of scFv, (scFv)₂, scFv-Fc, Fab, Fab' and F(ab')₂ of the anti-c-Met antibody.

5. The anti-c-Met antibody or antigen-binding fragment thereof according to claim 1, wherein the anti-c-Met antibody or antigen-binding fragment thereof crosslinks to human c-Met and mouse c-Met.

6. A bispecific antibody or antibody-drug conjugate comprising the anti-c-Met antibody or antigen-binding fragment thereof according to claim 1.

7. A pharmaceutical composition for preventing or treating cancer comprising the anti-c-Met antibody or antigen-binding fragment thereof according to claim 1 as an active ingredient.

8. A pharmaceutical composition for preventing or treating cancer comprising the bispecific antibody or antibody-drug conjugate according to claim 6 as an active ingredient.

9. The pharmaceutical composition according to claim 7 or 8, wherein the cancer is breast cancer, colon cancer, lung cancer, stomach cancer, liver cancer, blood cancer, bone cancer, pancreatic cancer, skin cancer, brain cancer, uterine cancer, nasopharyngeal cancer, laryngeal cancer, colon cancer, ovarian cancer, rectal cancer, colorectal cancer, vaginal cancer, small intestine cancer, endocrine cancer, thyroid cancer, parathyroid cancer, ureteral cancer, urethral cancer, prostate cancer, bronchial cancer, bladder cancer, kidney cancer or bone marrow cancer.

10. The pharmaceutical composition according to claim 7 or 8, wherein the pharmaceutical composition is used for combination therapy using radiation.

11. A nucleic acid encoding the anti-c-Met antibody or antigen-binding fragment thereof according to claim 1.

12. A recombinant expression vector comprising the nucleic acid according to claim 11.

13. A host cell transformed with the recombinant expression vector according to claim 12.

14. The host cell according to claim 13, wherein the host cell is selected from the group consisting of animal cells, plant cells, yeast, *Escherichia coli* and insect cells.

15. The host cell according to claim 13, wherein the host cell is selected from the group consisting of monkey kidney cells (COS7), NSO cells, SP2/0 cells, Chinese hamster ovary (CHO) cells, W138, baby hamster kidney (BHK) cells, MDCK, myeloma cell lines, HuT78 cells, HEK293 cells, *Bacillus subtilis, Streptomyces* sp., *Pseudomonas* sp., *Proteus mirabilis, Staphylococcus* sp., *Aspergillus* sp., *Pichia pastoris, Saccharomyces cerevisiae, Schizosaccharomyces* sp., and *Neurospora crassa.*

16. A method of producing an anti-c-Met antibody or antigen-binding fragment thereof comprising culturing the host cell according to any one of claims 13 to 15.

17. A composition for treating cancer by co-administration comprising the anti-c-Met antibody or antigen-binding fragment thereof according to claim 1 and an additional therapeutic agent for cancer.

18. The composition according to claim 17, wherein the therapeutic agent for cancer is an immune checkpoint inhibitor.

19. The composition according to claim 18, wherein the immune checkpoint inhibitor is an anti-CTLA-4 antibody, an anti-PD-1 antibody or an anti-PD-Ll antibody.

20. The composition according to claim 17, wherein the cancer is breast cancer, colon cancer, lung cancer, stomach cancer, liver cancer, blood cancer, bone cancer, pancreatic cancer, skin cancer, brain cancer, uterine cancer, nasopharyngeal cancer, laryngeal cancer, colon cancer, ovarian cancer, rectal cancer, colorectal cancer, vaginal cancer, small-intestine cancer, endocrine cancer, thyroid cancer, parathyroid cancer, ureteral cancer, urethral cancer, prostate cancer, bronchial cancer, bladder cancer, kidney cancer or bone-marrow cancer.
